(19) Europäisches Patentamt
European Patent Office
Office européen des brevets

(11) Publication number: **0 569 083 A1**

# (12) EUROPEAN PATENT APPLICATION

(21) Application number: **93201232.1**

(22) Date of filing: **29.04.93**

(51) Int. Cl.[5]: **C07D 239/80**, A61K 31/505, C07D 401/06, C07D 403/06, C07D 405/12, C07D 401/12, A61K 31/70

(30) Priority: **07.05.92 US 880119**
**16.12.92 US 991164**

(43) Date of publication of application:
**10.11.93 Bulletin 93/45**

(84) Designated Contracting States:
**AT BE CH DE DK ES FR GB GR IE IT LI LU NL PT SE**

(71) Applicant: **MERCK & CO. INC.**
**126, East Lincoln Avenue**
**P.O. Box 2000**
**Rahway New Jersey 07065-0900(US)**

(72) Inventor: **Lyle, Terry A.**
**570 Camp Wawa Road**
**Lederach, PA 19450(US)**
Inventor: **Tucker, Thomas J.**
**114 Station Drive**
**North Wales, PA 19454(US)**
Inventor: **Wiscount, Catherine M.**
**3096 Lindberg Avenue**
**Allentown, PA 18103(US)**

(74) Representative: **Ouillin, Helen Kaye et al**
**European Patent Department,**
**Merck & Co., Inc.,**
**Terlings Park,**
**Eastwick Road**
**Harlow, Essex CM20 2OR (GB)**

## (54) New quinazolines as inhibitors of HIV reverse transcriptase.

(57) Compounds having a quinazolin-2-one nucleus with a substituted alkynyl or substituted alkenyl at the 4-position are described. These compounds are useful in the inhibition of HIV reverse transcriptase (including its resistant varieties), the prevention or treatment of infection by HIV and the treatment of AIDS, either as compounds, pharmaceutically acceptable salts, pharmaceutical composition ingredients, whether or not in combination with other antivirals, immunomodulators, antibiotics or vaccines. Methods of treating AIDS and methods of preventing or treating infection by HIV are also described.

EP 0 569 083 A1

## BACKGROUND OF THE INVENTION

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is reverse transcription of the RNA genome by a virally encoded reverse transcriptase to generate DNA copies of HIV sequences, a required step in viral replication. It is known that some compounds are reverse transcriptase inhibitors and are effective agents in the treatment of AIDS and similar diseases, e.g., azidothymidine or AZT.

Nucleotide sequencing of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267 (1985); Power, M.D. et al., Science, 231, 1567 (1986); Pearl, L.H. et al., Nature 329, 351 (1987)].

Applicants demonstrate that the compounds of this invention are inhibitors of HIV reverse transcriptase. The particular advantages of the present compounds are their demonstrated inhibition of resistant HIV reverse transcriptase.

## BRIEF DESCRIPTION OF THE INVENTION

Compounds of formula I, as herein defined, are disclosed. These compounds are useful in the inhibition of HIV reverse transcriptase (and its resistant varieties), the prevention of infection by HIV, the treatment of infection by HIV and in the treatment of AIDS and/or ARC, either as compounds, pharmaceutically acceptable salts (when appropriate), pharmaceutical composition ingredients, whether or not in combination with other antivirals, anti-infectives, immunomodulators, antibiotics or vaccines. Methods of treating AIDS, methods of preventing infection by HIV, and methods of treating infection by HIV are also disclosed.

## DETAILED DESCRIPTION OF THE INVENTION AND PREFERRED EMBODIMENTS

This invention is concerned with compounds of formula I, combinations thereof, or pharmaceutically acceptable salts thereof, in the inhibition of HIV reverse transcriptase and its resistant varieties, the prevention or treatment of infection by HIV and in the treatment of the resulting acquired immune deficiency syndrome (AIDS). Compounds of formula I are defined as follows:

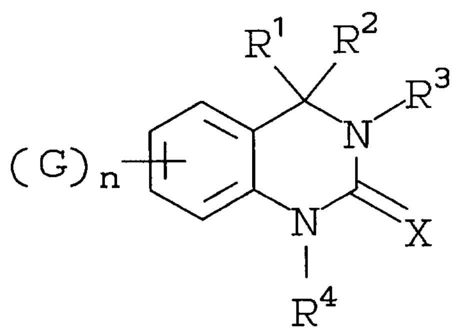

I

wherein

X is O;

G, when present is halo, nitro, or cyano;

n is 0-4;

$R^1$ is $C_{3-5}$cycloalkyl, $C_{2-5}$alkynyl, $C_{2-4}$alkenyl, or cyano;

$R^2$ is $C_{2-5}$alkynyl substituted with one or more of A, or $C_{2-5}$alkenyl substituted with one or more of A,

wherein

A is

i) halo,

ii) hydroxy,

iii) amino,

iv) cyano,
v) nitro,
vi) azido,
vii) $C_{3-8}$cycloalkyl,
viii) $C_{1-4}$alkoxy, unsubstituted or substituted with one or more of halo,
ix) di-($C_{1-4}$alkyl)amino,
x) $C_{1-4}$alkylamino,
xi) aryl, unsubstituted or substituted with one or more of D, wherein D is amino, nitro, cyano, or $C_{1-3}$alkoxy,
xii) aryloxy, unsubstituted or substituted with one or more of D;
xiii) heterocycle, unsubstituted or substituted with one or more of D;
xiv) heterocycle oxy; or
xv) $C_{2-5}$alkenyl;
xvi) COOR, wherein R is H, $C_{1-4}$alkyl or aryl;
xvii) $CONR_2$; or
xviii) COR;

$R^3$ is
i) H;
ii) cyano;
iii) amino;
iv) hydroxyl;
v) $C_{1-4}$alkyl, unsubstituted or substituted with one or more of E, wherein E is halo, hydroxyl, amino, nitro, cyano, $C_{1-4}$alkoxy, or $C_{3-5}$cycloalkyl;
vi) $C_{2-4}$alkenyl, unsubstituted or substituted with E; or
vii) $C_{2-4}$alkynyl, unsubstituted or substituted with E;

$R^4$ is
i) H;
ii) $C_{1-4}$alkyl;
iii) $C_{1-5}$alkylcarbonyl;
iv) benzoyl, unsubstituted or substituted with one or more of A; or
v) heterocyclecarbonyl;

with the proviso that any terminal alkynyl carbon is not substituted with any substituent selected from the group consisting of halo, hydroxy, amino, cyano, nitro, azido, $C_{1-4}$alkoxy unsubstituted or substituted with one or more of halo, di-($C_{1-4}$alkyl)amino, $C_{1-4}$alkylamino, aryloxy unsubstituted or substituted with one or more of D, or heterocycle oxy;
or a pharmaceutically acceptable salt thereof.

In one embodiment, compounds further are limited to formula II:

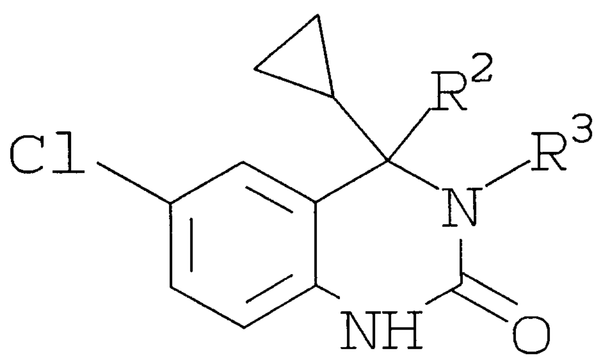

II

wherein:

$R^2$ is $C_{2-5}$ alkynyl substituted with halo, hydroxy, amino, cyano, nitro, azido, $C_{3-8}$ cycloalkyl, $C_{1-4}$ alkoxy, di-($C_{1-4}$alkyl)amino, $C_{1-4}$alkylamino,
phenyl, 2-nitrophenyl, pyridyl, pyrimidyl, pyrazinyl, imidazolyl, or $C_{2-3}$alkenyl;

$R^3$ is H or $C_{1-3}$ alkyl;

or a pharmaceutically acceptable salt thereof.

Specific illustrations of the compounds of this invention include those of the following Table.

TABLE

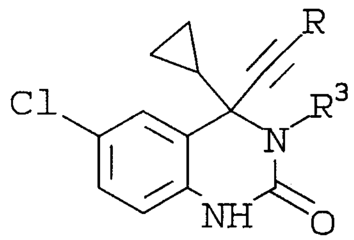

| Compound | Ex. | $R^3$ | R | $IC_{50}$ | $IC_{50}$DBL Mutant |
|---|---|---|---|---|---|
| 1 | 4 | H | $CH_2OCH_3$ | 49 nM | 7300 nM |
| 2 | 3 | $CH_3$ | $CH_2OCH_3$ | 13 nM | 700 nM |
| 3 | 20 | $CH_3$ | $CH_2OCH_2CF_3$ | 19 nM | 2600 nM |
| 4 | 13 | $CH_3$ | $CH_2CH_2F$ | 7 nM | 230 nM |
| 5 | 16 | H | $CH_2CH_2F$ | 31 nM | 3500 nM |
| 6 | 14 | $CH_3$ | $CH_2CH_2Cl$ | 7 nM | 250 nM |
| 7 | 18 | $CH_3$ | $CH_2N_3$ | 20 nM | 1750 nM |
| 8 | 17 | $CH_3$ | $CH_2F$ | 24 nM | 2900 nM |
| 9 | 33 | $CH_3$ | $CH_2N(CH_3)_2$ | 54 nM | 273 nM |
| 10 | 29 | $CH_3$ | 2-pyridyl | 21 nM | 87 nM |
| 11 | 24 | H | 2-pyridyl ($^+/-$) | 19 nM | 635 nM |

| Compound | Ex. | $R^3$ | R | $IC_{50}$ | $IC_{50}$ DBL Mutant |
|---|---|---|---|---|---|
| 12 | 25 | H | 3-pyridyl | 84 nM | 310 nM |
| 13 | 26 | H | 4-pyridyl | 470 nM | 3000 nM |
| 14 | 28 | H | 5-pyrimidyl | 1750 nM | 3000 nM |
| 15 | 35 | H | 2-pyrimidyl | 130 nM | 8800 nM |
| 16 | 27 | H | 2-pyrazinyl | 270 nM | 9000 nM |
| 17 | 21 | $CH_3$ | $CH_2O$-4-pyridyl | 165 nM | 3900 nM |
| 18 | 22 | $CH_3$ | $CH_2O$-4-pyridyl-N-oxide | 9400nM | 65000 nM |
| 19 | 9 | $CH_3$ | $CH_2$(4-morpholinyl) | 1000 nM | 22000 nM |
| 20 | 19 | $CH_3$ | $CH_2$(1-imidazolyl) | 620 nM | 960 nM |
| 21 | 35 | $CH_3$ | $CH=CH_2$ | 4.3 nM | 248 nM |
| 22 | 36 | $CH_3$ | $CH_2OH$ | 255 nM | |
| 23 | 34 | H | phenyl | 6.1 nM | 135 nM |
| 24 | 37 | $CH_3$ | $CH_2O$-2-pyridyl | 125 nM | 4900 nM |
| 25 | 38 | H | 2-nitrophenyl | 10.5 nM | 390 nM |
| 26 | 40 | H | 2-pyridyl (-) | 7 nM | 400 nM |

Preferred compounds include
6-chloro-4-cyclopropyl-4-(4-fluoro-1-butynyl)-3, 4-dihydro-3-methylquinazolin-2(1H)-one (Compound 4),
6-chloro-4-cyclopropyl-3,4-dihydro-3-methyl-4-((2-pyridyl)ethynyl)quinazolin-2(1H)-one (Compound 10),
6-chloro-4-cyclopropyl-3,4-dihydro-4-((2-pyridyl)ethynyl)quinazolin-2(1H)-one (Compound 11),
6-chloro-4-cyclopropyl-3,4-dihydro-4-(phenylethynyl)quinazolin-2(1H)-one (Compound 23), or
(-)6-chloro-4-cyclopropyl-3,4-dihydro-4-((2-pyridyl)ethynyl)quinazolin-2(1H)-one(Compound 26)
or a pharmaceutically acceptable salt thereof.

Compound 26 has (S) stereochemistry at the 4-position, with the structure:

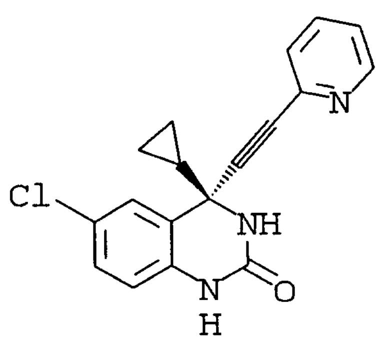

6-chloro-4(S)-cyclopropyl-3,4-dihydro-4-((2-pyridyl)ethynyl)quinazolin-2(1H)-one

The compounds of the present invention may have asymmetric centers and may occur, except when specifically noted, as racemates, racemic mixtures or as individual diastereomers, or enantiomers, with all isomeric forms being included in the present invention.

When any variable (e.g., G, $R^1$, $R^2$, $R^3$, etc.) occurs more than one time in any constituent or in formula I, its definition on each occurrence is independent of its definition at every other occurrence. Also, combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms; "alkenyl" is intended to cover both branched- and straight chain alkyl groups with at least one carbon-carbon double bond; "alkynyl" is intended to cover both branched- and straight chain alkyl groups with at least one carbon-carbon triple bond. "Halogen" or "halo" as used herein, means fluoro, chloro, bromo and iodo.

As used herein, with exceptions as noted, "aryl" is intended to mean phenyl, naphthyl, tetrahydronaphthyl, biphenyl, phenanthryl, anthryl or acenaphthyl.

The term heterocycle or heterocyclic, as used herein except where noted, represents a stable 5- to 7-membered monocyclic or stable 8- to 11-membered bicyclic heterocyclic ring which is either saturated, partially unsaturated or unsaturated, and which consists of carbon atoms and from one to four heteroatoms selected from the group consisting of N, O and S, and wherein the nitrogen and sulfur heteroatoms may optionally be oxidized, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include piperidinyl, piperazinyl, 2-oxopiperazinyl, 2-oxopiperidinyl, 2-oxopyrrolidinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, benzofuranyl, tetrahydropyranyl, thienyl, benzothienyl, thiamorpholinyl, thiamorpholinyl sulfoxide, thiamorpholinyl sulfone, and oxadiazolyl.

The compounds of the present invention can be synthesized by the following methods.

METHOD A

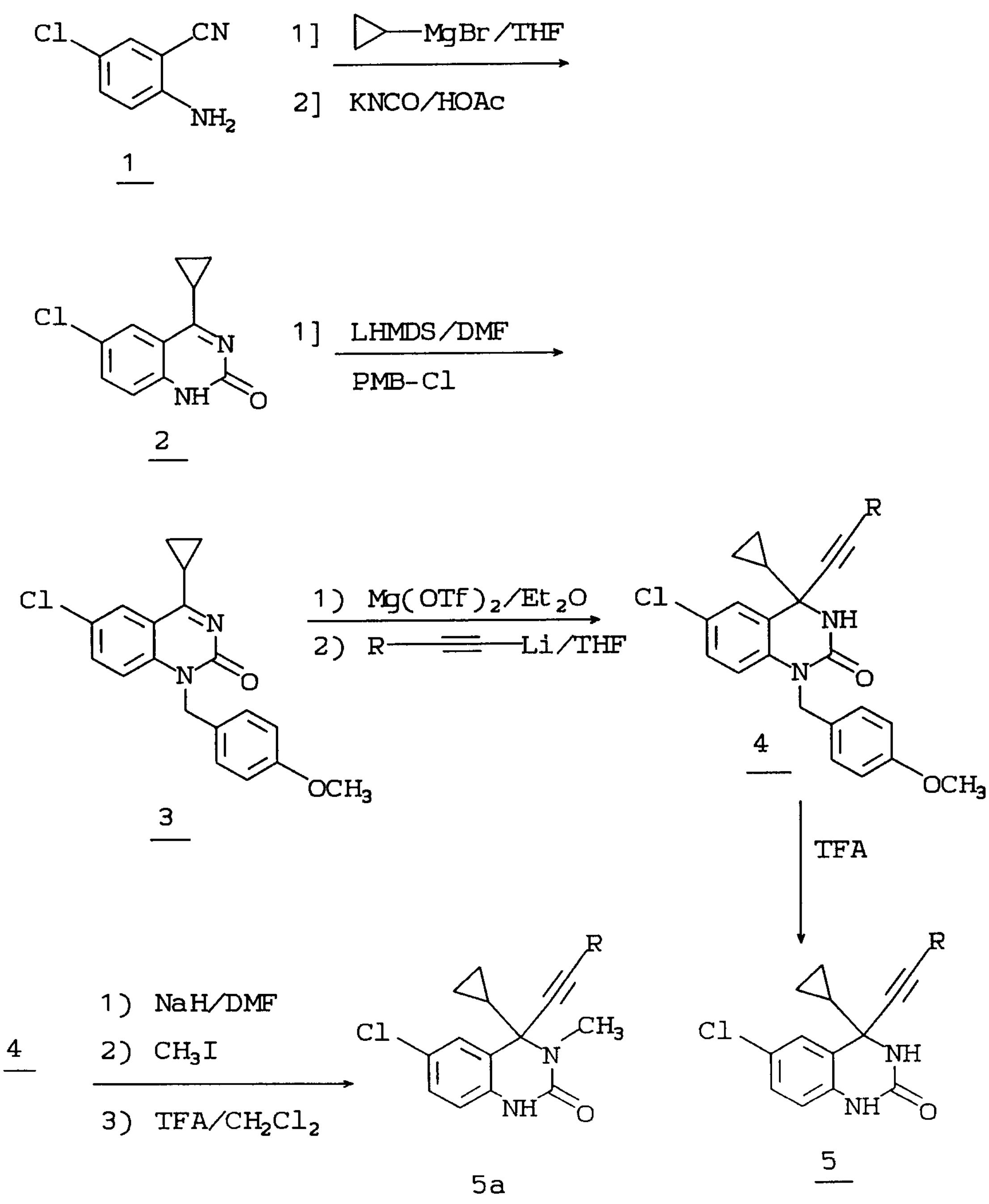


1

2

3

4

4

5a

5

The characteristic feature of Method A is R-metal addition to a dihydroquinazoline in the presence of magnesium ions and other good Lewis acids. Method A is further illustrated by Examples 24-29.

## METHOD B

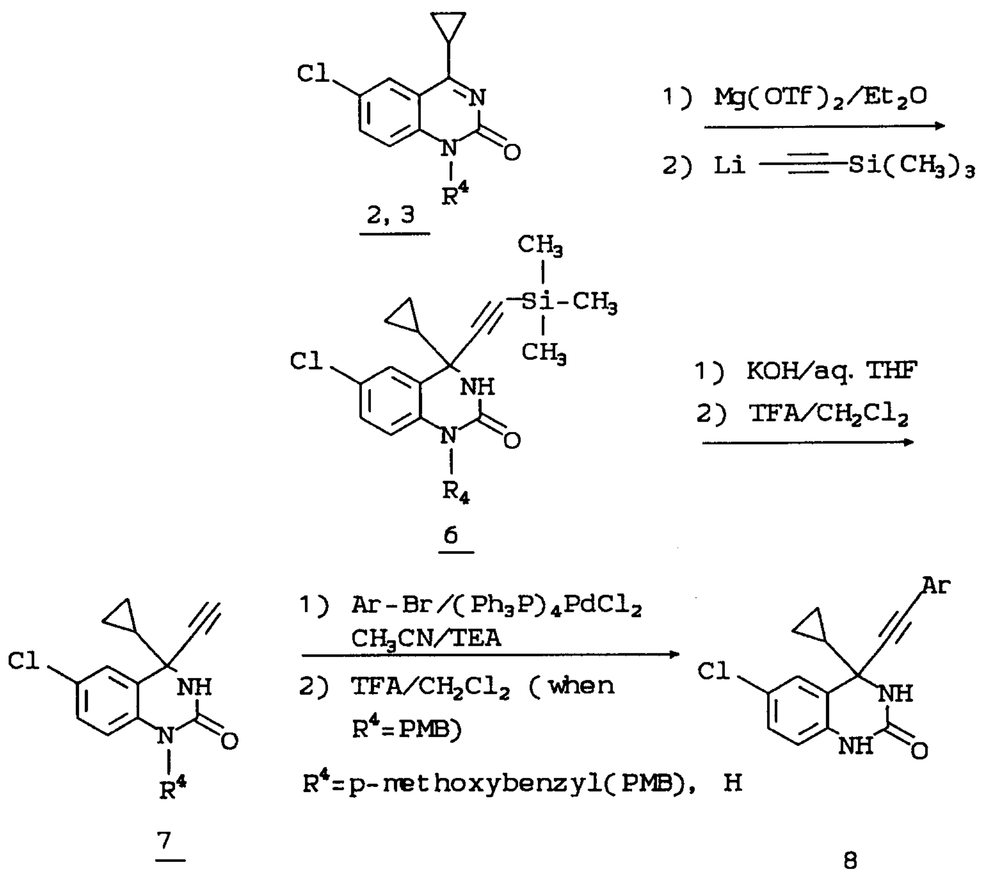


2, 3

6

7

8

Method B involves a cross-coupling reaction in the presence of palladium (II) chloride-triphenylphosphine couplex as a catalyst, to give aryl and heterocyclic substitutions of the 4-acetylene group. Example 35 illustrates the Method.

## METHOD C

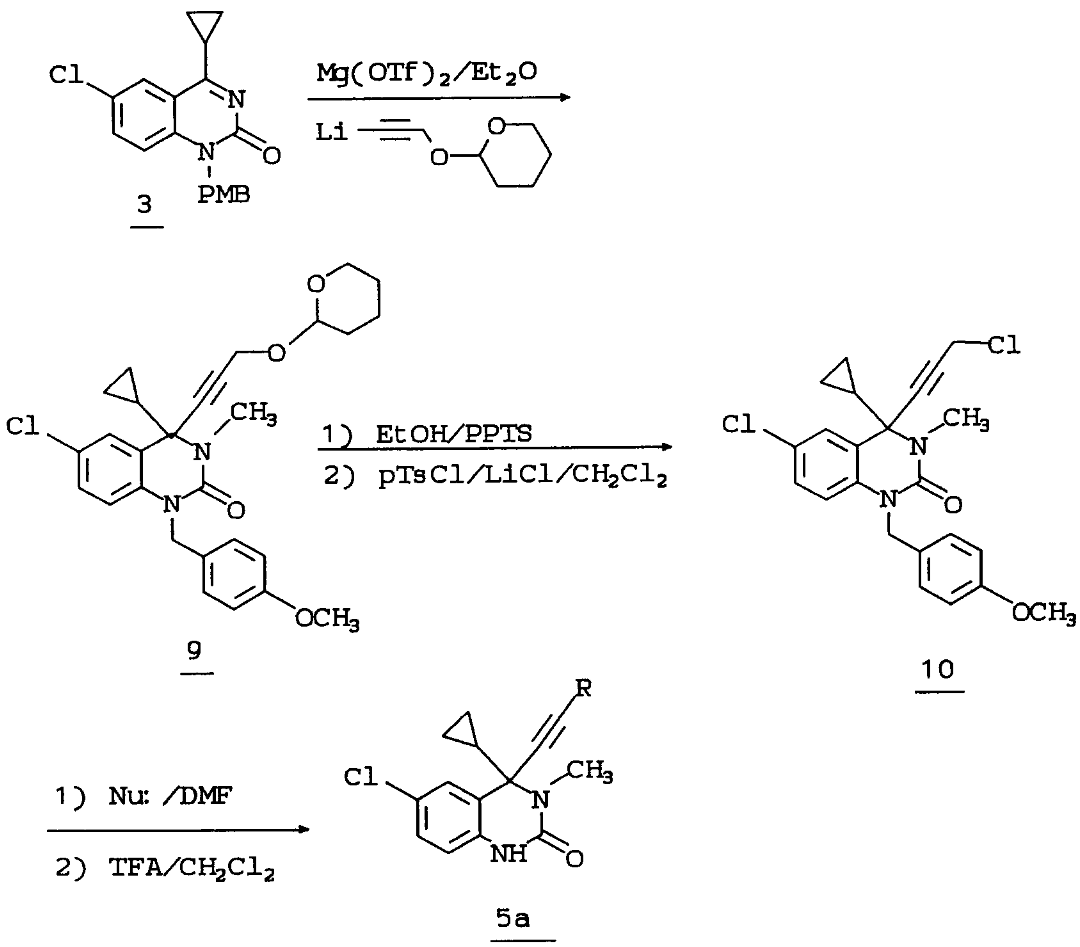


3

9

10

5a

Method C depicts another method of obtaining substituted 4-acetylene derivatives. A tetrahydropyran derivative 9 is formed by R-metal addition as in Method A, followed by reaction with an alcohol in the presence of pyridinium paratoluene sulfonate (PPTS) to form the corresponding alcohol intermediate 11, wherein $R^3$ is $CH_3$. Chlorination, followed by nucleophilic substitution with the desired end group gives the appropriate product 5a. Method C is specifically illustrated by Examples 5-9.

METHOD D

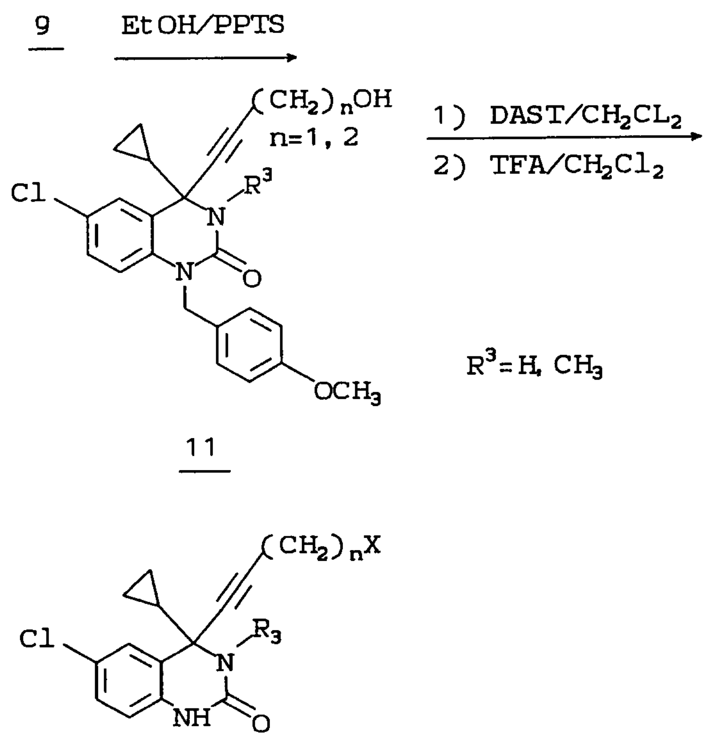


9

11

x = halo

12

Method D is suitable for halo substituted 4-alkynyl derivatives. The penultimate hydroxy derivative 11 is formed as in Method C, followed by reaction with the florinating agent diethylaminosulfurtrifluoride (DAST). Deprotection may then be desired. Chlorination is a side reaction. Method D is specifically illustrated by Examples 11-14.

The compounds of the present inventions are useful in the inhibition of HIV reverse transcriptase, the prevention of treatment of infection by human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymptomatic, and actual or potential exposure to HIV. For example, the compounds of this invention are useful in treating infection by HIV after suspected past exposure to HIV by e.g., blood transfusion, exchange of body fluids, bites, accidental needle stick, or exposure to patient blood during surgery.

The particular advantage of the compounds of this invention is their potent inhibition against HIV reverse transcriptase rendered resistant to other antivirals, such as L-697,661, which is 3-([(4,7-dichloro-1,3-benzoxazol-2-yl)methyl]-amino)-5-ethyl-6-methyl-pyridin-2(1H)-one; or L-696,229, which is 3-[2-(1,3-benzoxazol-2-yl)ethyl]-5-ethyl-6-methylpyridin-2(1H)-one; or AZT.

The compounds of this invention are also useful in the preparation and execution of screening assays for antiviral compounds. For example, the compounds of this invention are useful for isolating enzyme mutants, which are excellent screening tools for more powerful antiviral compounds. Furthermore, the compounds of this invention are useful in establishing or determining the binding site of other antivirals to HIV reverse transcriptase, e.g., by competitive inhibition. Thus the compounds of this invention are commercial products to be sold for these purposes.

For these purposes, the compounds of the present invention may be administered orally, parenterally (including subcutaneous injections, intravenous, intramuscular, intrasternal injection or infusion techniques), by inhalation spray, or rectally, in dosage unit formulations containing conventional non-toxic

pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically-effective amount of a compound of the present invention.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

The compounds of this invention can be administered orally to humans in a dosage range of 0.1 to 100 mg/kg body weight in divided doses. One preferred dosage range is 0.1 to 10 mg/kg body weight orally in divided doses. Another preferred dosage range is 0.1 to 20 mg/kg body weight orally in divided doses. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The present invention is also directed to combinations of the HIV reverse transcriptase inhibitor compounds with one or more agents useful in the treatment of AIDS. For example, the compounds of this invention may be effectively administered, whether at periods of pre-exposure and/or post-exposure, in combination with effective amounts of the AIDS antivirals, immunomodulators, antiinfectives, or vaccines, such as those in the following Table C.

TABLE C

ANTIVIRALS

| Drug Name | Manufacturer | Indication |
| --- | --- | --- |
| AL-721 | Ethigen (Los Angeles, CA) | ARC, PGL HIV positive, AIDS |
| Recombinant Human Interferon Beta | Triton Biosciences (Almeda, CA) | AIDS, Kaposi's sarcoma, ARC |
| Acemannan | Carrington Labs (Irving, TX) | ARC (See also immunomodulators) |
| Cytovene Ganciclovir | Syntex (Palo Alto, CA) | sight threatening CMV peripheral CMV retinitis |
| d4T Didehydrodeoxy-thymidine | Bristol-Myers (New York, NY) | AIDS, ARC |
| ddI Dideoxyinosine | Bristol-Myers (New York, NY) | AIDS, ARC |
| EL10 | Elan Corp, PLC (Gainesville, GA) | HIV infection (See also immunomodulators) |

| Drug Name | Manufacturer | Indication |
|---|---|---|
| Trisodium Phosphonoformate | Astra Pharm. Products, Inc. (Westborough, MA) | CMV retinitis, HIV infection, other CMV infections |
| Dideoxycytidine; ddC | Hoffman-La Roche (Nutley, NJ) | AIDS, ARC |
| Novapren | Novaferon Labs, Inc. (Akron, OH) Diapren, Inc. (Roseville, MN, marketer) | HIV inhibitor |
| Peptide T Octapeptide Sequence | Peninsula Labs (Belmont, CA) | AIDS |
| Zidovudine; AZT | Burroughs Wellcome (Rsch. Triangle Park, NC) | AIDS, adv, ARC pediatric AIDS, Kaposi's sarcoma, asymptomatic HIV infection, less severe HIV disease, neurological involvement, in combination with other therapies. |

| Drug Name | Manufacturer | Indication |
|---|---|---|
| Ansamycin LM 427 | Adria Laboratories (Dublin, OH) Erbamont (Stamford, CT) | ARC |
| Dextran Sulfate | Ueno Fine Chem. Ind. Ltd. (Osaka, Japan) | AIDS, ARC, HIV positive asymptomatic |
| Virazole Ribavirin | Viratek/ICN (Costa Mesa, CA) | asymptomatic HIV positive, LAS, ARC |
| Alpha Interferon | Burroughs Wellcome (Rsch. Triangle Park, NC) | Kaposi's sarcoma, HIV in combination w/Retrovir |
| Acyclovir | Burroughs Wellcome | AIDS, ARC, asymptomatic HIV positive, in combination with AZT. |
| Antibody which neutralizes pH labile alpha aberrant Interferon in an immuno-adsorption column | Advanced Biotherapy Concepts (Rockville, MD) | AIDS, ARC |

| Drug Name | Manufacturer | Indication |
| --- | --- | --- |
| L-697,661 | Merck (Rahway, NJ) | AIDS, ARC, asymptomatic HIV positive, also in combination with AZT. |
| L-696,229 | Merck (Rahway, NJ) | AIDS, ARC, asymptomatic HIV positive, also in combination with AZT. |
| L-735,524 | Merck (Rahway, NJ) | AIDS, ARC, asymptomatic HIV positive, also in combination with AZT. |

IMMUNO-MODULATORS

| Drug Name | Manufacturer | Indication |
|---|---|---|
| AS-101 | Wyeth-Ayerst Labs. (Philadelphia, PA) | AIDS |
| Bropirimine | Upjohn (Kalamazoo, MI) | advanced AIDS |
| Acemannan | Carrington Labs, Inc. (Irving, TX) | AIDS, ARC (See also anti-virals) |
| CL246,738 | American Cyanamid (Pearl River, NY) Lederle Labs (Wayne, NJ) | AIDS, Kaposi's sarcoma |
| EL10 | Elan Corp, PLC (Gainesville, GA) | HIV infection (See also anti-virals) |
| Gamma Interferon | Genentech (S. San Francisco, CA) | ARC, in combination w/TNF (tumor necrosis factor) |
| Granulocyte Macrophage Colony Stimulating Factor | Genetics Institute (Cambridge, MA) Sandoz (East Hanover, NJ) | AIDS |

| Drug Name | Manufacturer | Indication |
|---|---|---|
| Granulocyte Macrophage Colony Stimulating Factor | Hoeschst-Roussel (Somerville, NJ) Immunex (Seattle, WA) | AIDS |
| Granulocyte Macrophage Colony Stimulating Factor | Schering-Plough (Madison, NJ) | AIDS<br>AIDS, in combination w/AZT |
| HIV Core Particle Immunostimulant | Rorer (Ft. Washington, PA) | seropositive HIV |
| IL-2 Interleukin-2 | Cetus (Emeryville, CA) | AIDS, in combination w/AZT |
| IL-2 Interleukin-2 | Hoffman-La Roche (Nutley, NJ) Immunex | AIDS, ARC, HIV, in combination w/AZT |
| Immune Globulin Intravenous (human) | Cutter Biological (Berkeley, CA) | pediatric AIDS, in combination w/AZT |
| IMREG-1 | Imreg (New Orleans, LA) | AIDS, Kaposi's sarcoma, ARC, PGL |
| IMREG-2 | Imreg (New Orleans, LA) | AIDS, Kaposi's sarcoma, ARC, PGL |

| Drug Name | Manufacturer | Indication |
| --- | --- | --- |
| Imuthiol Diethyl Dithio Carbamate | Merieux Institute (Miami, FL) | AIDS, ARC |
| Alpha-2 Interferon | Schering Plough (Madison, NJ) | Kaposi's sarcoma w/AZT: AIDS |
| Methionine-Enkephalin | TNI Pharmaceutical (Chicago, IL) | AIDS, ARC |
| MTP-PE Muramyl-Tripeptide | Ciba-Geigy Corp. (Summit, NJ) | Kaposi's sarcoma |
| Granulocyte Colony Stimulating Factor | Amgen (Thousand Oaks, CA) | AIDS, in combination w/AZT |
| rCD4 Recombinant Soluble Human CD4 | Genentech (S. San Francisco, CA) | AIDS, ARC |
| rCD4-IgG hybrids | | AIDS, ARC |
| Recombinant Soluble Human CD4 | Biogen (Cambridge, MA) | AIDS, ARC |
| Interferon Alfa 2a | Hoffman-La Roche (Nutley, NJ) | Kaposi's sarcoma AIDS, ARC, in combination w/AZT |

| Drug Name | Manufacturer | Indication |
|---|---|---|
| SK&F106528 Soluble T4 | Smith, Kline & French Laboratories (Philadelphia, PA) | HIV infection |
| Thymopentin | Immunobiology Research Institute (Annandale, NJ) | HIV infection |
| Tumor Necrosis Factor; TNF | Genentech (S. San Francisco, CA) | ARC, in combination w/gamma Interferon |

ANTI-INFECTIVES

| Drug Name | Manufacturer | Indication |
|---|---|---|
| Clindamycin with Primaquine | Upjohn (Kalamazoo, MI) | PCP |
| Fluconazole | Pfizer (New York, NY) | cryptococcal meningitis, candidiasis |
| Pastille Nystatin Pastille | Squibb Corp. (Princeton, NJ) | prevention of oral candidiasis |
| Ornidyl Eflornithine | Merrell Dow (Cincinnati, OH) | PCP |
| Pentamidine Isethionate (IM & IV) | LyphoMed (Rosemont, IL) | PCP treatment |

| Drug Name | Manufacturer | Indication |
|---|---|---|
| Piritrexim | Burroughs Wellcome (Rsch. Triangle Park, NC) | PCP treatment |
| Pentamidine isethionate for inhalation | Fisons Corporation (Bedford, MA) | PCP prophylaxis |
| Spiramycin | Rhone-Poulenc Pharmaceuticals (Princeton, NJ) | cryptosporidial diarrhea |
| Intraconazole-R51211 | Janssen Pharm. (Piscataway, NJ) | histoplasmosis; cryptococcal meningitis |
| Trimetrexate | Warner-Lambert | PCP |

OTHER

| Drug Name | Manufacturer | Indication |
|---|---|---|
| Recombinant Human Erythropoietin | Ortho Pharm. Corp. (Raritan, NJ) | severe anemia assoc. with AZT therapy |
| Megestrol Acetate | Bristol-Myers (New York, NY) | treatment of anorexia assoc. w/AIDS |
| Total Enteral Nutrition | Norwich Eaton Pharmaceuticals (Norwich, NY) | diarrhea and malabsorption related to AIDS |

It will be understood that the scope of combinations of the compounds of this invention with AIDS antivirals, immunomodulators, anti-infectives or vaccines is not limited to the list in the above Table, but includes in principle any combination with any pharmaceutical composition useful for the treatment of AIDS. The compound L-735,524 is an HIV protease inhibitor with the chemical name N-(2(R)-hydroxy-1(S)-indanyl)-2(R)-phenylmethyl-4-(S)-hydroxy-5-(1-(4-(3-pyridyl-methyl)-2(S)-N'-(t-butylcarboxamido)piperazinyl)-)-pentaneamide.

EXAMPLE 1

6-chloro-4-cyclopropyl-3,4-dihydro-1-(4-methoxybenzyl)-4-(3-methoxy-1-propynyl)quinazolin-2(1H)-one

A. (2-Amino-4-chlorophenyl)cyclopropyl ketone

A solution of cyclopropylmagnesium bromide, prepared from 2.4 g (0.099 g atom) of magnesium turnings and 13.0 g (0.107 mol) of cyclopropyl bromide in 100 mL of THF, was stirred at 38 °C as a solution of 5-chloro-anthranilonitrile (3.65 g, 0.0239 mol) in 40 mL THF was added over 20 min. Stirring was continued at 40 °C for 2h, following which the reaction mixture was cooled in an ice bath and 50 mL of saturated $NH_4Cl$ was added, followed by 100 mL of 2N HCl. The cooling bath was removed and stirring was continued at room temperature for 2h. The mixture was then brought to pH 9 by addition of 20% NaOH and was extracted 3 times with ether. The combined organic phases were washed with brine and dried over $MgSO_4$. Following removal of the solvents the oily residue was flash chromatographed, eluting with 15% EtOAc in hexane, to provide 3.0 g (64%) of the title compound as a yellow solid, MP 66-68 °C.
$^1$H NMR ($CDCl_3$): δ 1.02 (m, 2H), 1.18 (m, 2H), 2.57 (m, 1H), 6.17 (s, 1H), 6.61 (d, J = 8.5 Hz, 1H), 7.22 (dd, J = 2, 8.5 Hz, 1H), 7.92 (d, J = 2 Hz, 1H).

B. 6-chloro-4-cyclopropylquinazolin-2(1H)-one.

To a stirred suspension of 150 mg (0.767 mmol) of the product from Step A in 3 mL of glacial acetic acid at 0° was added a solution of 75 mg (0.922 mmol) of potassium cyanate in 0.3 mL of $H_20$ in one portion. After stirring for 1 hour at 0°-5°, the reaction mixture was allowed to warm to room temperature over a 1 hour period. The reaction mixture was partitioned between EtOAc and $H_2O$, the organic layer washed with $H_20$, filtered, washed with brine, dried over $Na_2SO_4$, and concentrated to afford 120 mg of a light yellow solid. This material was chromatographed on silica gel to give 122 mg of the title compound as a solid:
$^1$H-NMR ($CDCl_3$): δ 1.27 (m, 2H), 1.57 (m, 2H), 2.55 (m, 1H), 7.48 (d, J = 8 Hz, 1H), 7.61 (dd, J = 8, 2 Hz, 1H), 8.10 (d, J = 2 Hz, 1H). FAB MS M + H = 221, mp = 215-217 °C.

C. 6-chloro-4-cyclopropyl-1-(4-methoxybenzyl) quinazolin-2(1H)-one

To a stirred solution of 75 mg (0.338 mmol) of the product from Step B in 6 mL of dry DMF was added 17 mg (0.423 mmol) of sodium hydride (60% in mineral oil) in one portion. After 20 minutes when gas evolution ceased, 50 µL (0.372 mmol) of 4-methoxybenzylchloride was added in one portion. The reaction solution was stirred at room temperature for 2.5 hours, then heated to 80° under Ar for 4 hours, and allowed to stir at room temperature for 2.5 days. The reaction mixture was concentrated at reduced pressure and the residue partitioned between EtOAc and $H_2O$. The organic layer was washed with water, brine, dried over $Na_2SO_4$, and concentrated to give a residue which was chromatographed on silica gel using 1:1 EtOAc-hexane to give 73 mg of the title compound. An analytical sample was obtained by crystallization from EtOAc-hexane:
$^1$H-NMR ($CDCl_3$): δ 1.25 (m, 2H), 1.56 (m, 2H), 2.52 (m, 1H), 3.76 (s, 3H), 5.40 (s, 2H), 6.83 (d, J = 8.7 Hz, 2H), 7.18 (d, J = 8.7 Hz, 2H), 7.21 (d, J = 9.2 Hz, 1H), 7.53 (dd, J = 2.3, 9.2 Hz, 1H), 8.1 (d, J = 2.3Hz, 1H). FAB MS M + H = 341, mp 211-213 °C.

D. 6-chloro-4-cyclopropyl-3,4-dihydro-1-(4-methoxybenzyl)-4-(3-methoxy-1-propynyl)quinazolin-2(1H)-one

A suspension of 548 mg (1.61 mmol) of 6-chloro-4-cyclopropyl-1-(4-methyoxybenzyl)-quinazolin-2 (1H)-one and 1.55 g (4.82 mmol) of magnesium triflate was stirred for 30 min in 18 mL of ether under Ar at rt. To this solution was added a -78° solution of 1-lithio-3-methoxypropyne (prepared by adding 543 µL (6.4 mmol) of 3-methoxy-1-propyne dropwise to a -78° solution of 6.4 mmol of lithiumdiisoproylamide in 18 mL of THF under Ar) via cannula. After 2 h at rt, the reaction was quenched by pouring into ice-cold 1M citric acid and extracted with two portions of $CHCl_3$. The organic layers were washed with 10% $Na_2CO_3$, dried over $MgSO_4$, treated with activated carbon and solvents removed to give 662 mg of an amber foam:
NMR ($CDCl_3$): δ 0.55-0.72(m, 2H), 0.74-0.90(m, 2H), 1.38-1.50(m, 1H), 3.32(s, 3H), 3.76(s, 3H), 4.08(s, 2H), 5.02(d, J = 16.8 Hz, 1H), 5.19(d, J = 16.8 Hz, 1H), 5.36(s, 1H), 6.75(d, J = 9.0 Hz, 1H), 6.82(d, J = 9.0 Hz, 2H), 7.12(dd, J = 9.0, 2.4 Hz, 1H), 7.18(d, J = 9.0 Hz, 2H), 7.49(d, J = 2.4 Hz, 1H).

EXAMPLE 2

6-chloro-4-cyclopropyl-3,4-dihydro-3-methyl-1-(4-methoxybenzyl)-4-(3-methoxy-1-propynyl)quinazolin-2(1H)-one

A quantity of 130 mg (0.316 mmol) of the product from Example 1 was dissolved in 5 mL of dry DMF and treated with 25 mg (0.63 mmol) of 60% sodium hydride in oil under Ar. After stirring at room temp. for 40 min, 40 $\mu$L (0.63 mmol) of methyl iodide (dried by passing through a pad of alumina) was added in one portion via syringe, and the reaction mixture stirred overnight at room temp. The DMF was removed by rotovap and the residue partitioned between $CHCl_3$ and 1M citric acid. The aqueous layer was extracted with $CHCl_3$, and the combined organic layers washed with 10% $Na_2CO_3$, dried over $MgSO_4$, and solvents removed to afford 140 mg of a dark brown oil which was chromatographed on 13 g of fine $SiO_2$ using 98.5:1.5 to 90:10 $CHCl_3$-$CH_3CN$ to give 49 mg of the title compound as a pale yellow oil:
NMR ($CDCl_3$): $\delta$ 0.40-0.55(m, 2H), 0.66-0.90(m, 2H), 1.25-1.40(m, 1H), 3.29(s, 3H), 3.40(s, 3H), 3.76(s, 3H), 4.21(s, 2H), 4.98(d, J = 16.8 Hz, 1H), 5.21(d, J = 16.8 Hz, 1H), 6.70(d, J = 8.8 Hz, 1H), 6.83(d, J = 8.8 Hz, 2H), 7.10(dd, J = 8.8, 2.4 Hz, 1H), 7.165(d, J = 8.8 Hz, 2H), 7.47(d, J = 2.4 Hz, 1H).

EXAMPLE 3

6-chloro-4-cyclopropyl-3,4-dihydro-3-methyl-4-(3-methoxy-1-propynyl)quinazolin-2(1H)-one

A quantity of 49 mg (0.115 mmol) of the product from Example 2 was treated with a solution of 2.5 mL of trifluoroacetic acid in 3.5 mL of methylene chloride for 3 h under Ar. The solvents were removed by rotary evaporation and the residue partitioned between $CHCl_3$ and 10% $Na_2CO_3$. The organic layer was dried over $Na_2SO_4$ and the solvents removed to give 47 mg of a yellow oil which was chromatographed on 5 g fine $SiO_2$ using 98:2 $CHCl_3$-$CH_3OH$ to afford 33 mg of the title compound which solidified upon lyophilization from dioxane: mp 119-121 °C,
NMR ($CDCl_3$): $\delta$ 0.40-0.55(m, 2H), 0.66-0.87(m, 2H), 1.32-1.42(m, 1H), 3.25(s, 3H), 3.41(s, 3H), 4.21(s, 2H), 6.77(d, J = 8.5 Hz, 1H), 7.17(dd, J = 8.5, 2.3 Hz, 1H), 7.38(d, J = 2.3 Hz, 1H), 9.23(s, 1H). Anal. Calc'd for $C_{16}H_{17}ClN_2O_2$ . $_{0.5\ H}$20 C 61.24 H 5.78 N 8.92 Found C 61.07 H 5.47 N 8.63

EXAMPLE 4

6-chloro-4-cyclopropyl-3,4-dihydro-4-(3-methoxy-1-propynyl)quinazolin-2(1H)-one

A quantity of 48 mg (0.116 mmol) of the product from Example 1 was treated according to the procedure of Example 3 above for 21 h to give a yellow oil which was chromatographed on 7 g fine $SiO_2$ using 97:3 $CHCl_3$-$CH_3OH$ to afford 12 mg of the title compound as an amorphous solid upon lyophilization from dioxane:
NMR ($CDCl_3$): $\delta$ 0.57-0.68(m, 2H), 0.74-0.88(m, 2H), 1.40-1.48(m, 1H), 3.32(s, 3H), 4.09(s, 2H), 5.23(s, 1H), 6.69(d, J = 8.5 Hz, 1H), 7.21(dd, J = 8.5, 2.3 Hz, 1H), 7.38(s, 1H), 7.45(d, J = 2.3 Hz, 1H), Anal. Calc'd for $C_{15}H_{15}ClN_2O_2$ . 0.3 dioxane C 61.35 H 5.53 N 8.83 Found C 61.05 H 5.24 N 8.75

EXAMPLE 5

6-chloro-4-cyclopropyl-3,4-dihydro-1-(4-methoxybenzyl)-4-(3-(tetrahydropyran-2-yl)oxy-1-propynyl)-quinazolin-2(1H)-one

A suspension of 4.8 g (14.1 mmol) of 6-chloro-4-cyclopropyl-1-(4-methyoxybenzyl)-quinazolin-2(1H-one (of Example 1, Step C) and 13.6 g (42.2 mmol) of magnesium triflate was stirred for 30 min in 125 mL of ether under Ar at rt. To this solution was added a -78° solution of 1-lithio-3-(tetrahydropyran-2-yl)-oxypropyne (prepared by adding 5.94 mL (42.2 mmol) of 3-(tetrahydropyran-2-yl)oxy-1-propyne dropwise to a -78° solution of 16.9 mL of 2.5M butyllithium in hexanes and 75 mL ether) via cannula. After stirring under Ar overnight at rt, two additional equivalents of 1-lithio-3-(tetrahydropyran-2-yl)oxypropyne (prepared as described above) were added to the reaction mixture. After 1h, the reaction was quenched by pouring into ice-cold 1M citric acid. The aqueous layer was extracted with EtOAc, and the combined organic layers were washed with. The organic layers were washed with 10% $NaHCO_3$, water, brine, dried over $Na_2SO_4$, and the solvents removed to give an oil which was chromatographed on fine $SiO_2$ using 1:2 EtOAc-hexane

to afford 4.51 g (67%) of the title compound as a colorless foam:
NMR ($CDCl_3$): δ 0.55-0.87(m, 4H), 1.40-1.85(m, 7H), 3.45-3.55(m, 1H), 3.73-3.85(m, 1H), 3.76(s, 3H), 4.25(s, 2H), 4.71(t, J = 2Hz, 1H), 5.11(dd, J=34.2, 16.8 Hz, 2H), 5.28(s, 1H), 6.735(d, J=9.0 Hz, 1H), 6.835(d, J=8.2 Hz, 2H), 7.10(dd, J=9.0, 2.4 Hz, 1H), 7.18(d, J=8.2 Hz, 2H), 7.47(d, J=2.4 Hz, 1H).

EXAMPLE 6

6-chloro-4-cyclopropyl-3,4-dihydro-3-methyl-1-(4-methoxybenzyl)-4-(3-(tetrahydropyran-2-yl)oxy-1-propynyl) quinazolin-2(1H)-one

A quantity of 4.1 g (8.52 mmol) of the product from Example 5 was treated by the procedure of Example 2 above to give 4.4 g of the title compound as an oil which was used in the subsequent step without further purification.

EXAMPLE 7

6-chloro-4-cyclopropyl-3,4-dihydro-3-methyl-1-(4-methoxybenzyl)-4-(3-hydroxy-1-propynyl)quinazolin-2(1H)-one

The product from Example 6 (4.22 g, 0.85 mmol) was dissoved in 75 mL of ethanol and treated with 214 mg (852 mmol) of pyridinium p-toluenesulfonate at 60°C under Ar for 5 h, followed by 17 h at room temp. The reaction mixture was concentrated and the residue partitioned between EtOAc and 10% $NaHCO_3$. The organic layer was washed with 10% $NaHCO_3$, water, brine, dried over $Na_2SO_4$ and solvents removed to give 3.73 g (quant.) of an off-white solid which was used in subsequent reactions without further purification.

EXAMPLE 8

6-chloro-4-cyclopropyl-3,4-dihydro-3-methyl-1-(4-methoxybenzyl)-4-(3-chloro-1-propynyl)quinazolin-2(1H)-one

To a solution of 1.25 g (3.04 mmol) of the product from Example 7 in 30 mL of $CH_2Cl_2$ was added 223 mg (1.82 mmol) of 4-(dimethylamino)pyridine, 696 mg (3.65 mmol) of p-toluenesulfonylchloride, 424 μL (3.04 mmol) of triethylamine, and 129 mg (3.04 mmol) of lithium chloride. After stirring at room temp. under Ar overnight, the reaction mixture was diluted with 75 mL of ether and filtered. The filtrate was washed with 10% $CuSO_4$, water, 10% $NaHCO_3$, brine, dried over $Na_2SO_4$ and solvents removed to give an oily solid which was chromatographed on fine $SiO_2$ using 1:12 EtOAc-$CHCl_3$ to afford 1.0 g (77%) of the title compound as a colorless solid:
NMR ($CDCl_3$): δ 0.40-0.57(m, 2H), 0.68-0.86(m, 2H), 1.31-1.38(m, 1H), 3.27(s, 3H), 3.76(s, 3H), 4.22(s, 2H), 4.97(d, J=16.2 Hz, 1H), 5.21(d, J=16.2 Hz, 1H), 6.71(d, J=8.8 Hz, 1H), 6.825(d, J=8.8 Hz, 2H), 7.11(dd, J=8.8, 2.4 Hz, 1H), 7.16(d, J=8.8 Hz, 2H), 7.44(d, J=2.4 Hz, 1H).

EXAMPLE 9

6-chloro-4-cyclopropyl-3,4-dihydro-3-methyl-4-(3-(4-morpholinyl)-1-propynyl)quinazolin-2(1H)-one

A solution of 50 mg (0.116 mmol) of the product from Example 8 in 1 mL of morpholine was stirred at room temp. under Ar for 1h, then stored in the freezer for 56 h. The solvent was removed by rotary evaporation, and the residue partitioned between water and EtOAc. The organic layer was washed with water, brine, dried over $Na_2SO_4$ and the solvents removed to give an oily solid which was treated by the procedure of Example 3 to afford 33 mg (78 %) of the title compound as a colorless solid: mp 157-160°C;
NMR ($CDCl_3$): δ 0.43-0.51(m, 2H), 0.66-0.84(m, 2H), 1.30-1.42(m, 1H), 2.59(t, J = 4.6 Hz, 4H), 3.25(s, 3H), 3.46(s, 2H), 3.76(t, J = 4.5 Hz, 4H), 6.76(d, J=8.5 Hz, 1H), 7.17(dd, J=8.5, 2.2 Hz, 1H), 7.379(d, J=2.2 Hz, 1H), 9.215(s, 1H).

EXAMPLE 10

6-chloro-4-cyclopropyl-3,4-dihydro-1-(4-methoxybenzyl)-4-(4-(tetrahydropyran-2-yl)oxy-1-butynyl)quinazolin-2 (1H)-one

A quantity of 200 mg (0.587 mmol) of 6-chloro-4-cyclopropyl-1-(4-methoxybenzyl)-quinazolin-2(1H)-one (of Example 1, Step C) was treated with 1-lithio-4-((tetrahydropyran-2-yl)oxy)butyne according to the procedure of Example 5 above to afford 281 mg (97 %) of the title compound as a colorless foam:
NMR ($CDCl_3$): $\delta$ 0.55-0.87(m, 4H), 1.40-1.85(m, 7H), 3.45-3.55(m, 1H), 3.73-3.85(m, 1H), 3.76(s, 3H), 4.25(s, 2H), 4.71(t, J = 2Hz, 1H), 5.11(dd, J=34.2, 16.8 Hz, 2H), 5.28(s, 1H), 6.735(d, J=9.0 Hz, 1H), 6.835(d, J=8.2 Hz, 2H), 7.10(dd, J=9.0, 2.4 Hz, 1H), 7.18(d, J=8.2 Hz, 2H), 7.47(d, J=2.4 Hz, 1H).

EXAMPLE 11

6-chloro-4-cyclopropyl-3,4-dihydro-1-(4-methoxybenzyl)-3-methyl-4-(4-(tetrahydropyran-2-yl)oxy-1-butynyl)-quinazolin-2(1H)-one

A quantity of 281 mg (0.568 mmol) of the product from Example 10 above was treated by the procedure of Example 2 above to afford 264 mg (91 %) of the title compound which was used in the subsequent reaction without further purification.

EXAMPLE 12

6-chloro-4-cyclopropyl-3,4-dihydro-1-(4-methoxybenzyl)-3-methyl-4-(4-hydroxy-1-butynyl)quinazolin-2(1H)-one

A quantity of 264 mg (0.519 mmol) of the product from Example 11 was treated by the procedure of Example 7 to afford 107 mg (48%) of the title compound which was used without further purification in the subsequent reaction.

EXAMPLE 13

6-chloro-4-cyclopropyl-4-(4-fluoro-1-butynyl)-3,4-dihydro-3-methylquinazolin-2(1H)-one

To a stirred solution of 107 mg (0.252 mmol) of the product from Example 12 in 2.0 mL of $CH_2Cl_2$ at 0 °C was added 100 $\mu$L (0.755 mmol) of diethylaminosulfurtrifluoride. The cold bath was removed, and the reaction stirred at room temp. for 2.5 h, poured into 20 mL of sat. $NaHCO_3$, and extracted with two portions of EtOAc. The combined organic layers were washed with water, brine, dried over $MgSO_4$ and solvents removed to give an oil which was chromatographed on 10 g of fine $SiO_2$ using 3:7 EtOAc-hexane to give 60 mg of a colorless oil which was treated by the procedure of Example 3 and purified by reversed phase HPLC using acetonitrile-0.1% aqueous trifluoroacetic acid on a C-18 column to give 29 mg (37 %) of the title compound as an amorphous solid:
NMR ($CDCl_3$): $\delta$ 0.39-0.47(m, 2H), 0.63-0.71(m, 1H), 0.76-0.84(m, 1H), 1.30-1.38(m, 1H), 2.72(dt, J = 21.4, 6.2 Hz, 2H), 3.23(s, 3H), 4.54(dt, J = 46.7, 6.2 Hz, 2H), 6.76(d, J=8.4 Hz, 1H), 7.168(dd, J=8.4, 2.4 Hz, 1H), 7.362(d, J=2.2 Hz, 1H), 9.2(s, 1H).

EXAMPLE 14

6-chloro-4-(4-chloro-1-butynyl)-4-cyclopropyl-3,4-dihydro-3-methylquinazolin-2(1H)-one

A quantity of 8 mg (10 %) of the title compound was isolated as a byproduct from Example 13 as an amorphous solid:
NMR ($CDCl_3$): $\delta$ 0.40-0.51(m, 2H), 0.64-0.72(m, 1H), 0.80-0.90(m, 1H), 1.30-1.38(m, 1H), 2.785(t, J = 6.6 Hz, 2H), 3.23(s, 3H), 3.65(t, J = 6.6 Hz, 2H), 6.68(d, J=8.4 Hz, 1H), 7.173(dd, J=8.4, 2.2 Hz, 1H), 7.42(d, J=2.2 Hz, 1H), 7.94(s, 1H).

EXAMPLE 15

6-chloro-4-cyclopropyl-3,4-dihydro-1-(4-methoxybenzyl)-4-(4-hydroxy-1-butynyl)quinazolin-2(1H)-one

A quantity of 75 mg (0.152 mmol) of the product from Example 10 was treated by the procedure of Example 7 to afford 61 mg (97%) of the title compound which was used without further purification in the subsequent reaction.

EXAMPLE 16

6-chloro-4-cyclopropyl-4-(4-fluoro-1-butynyl)-3,4-dihydroquinazolin-2(1H)-one

A quantity of 61 mg (0.148 mmol) of the product from Example 15 was treated by the procedure of Example 13 to give 7 mg (14 %) of the title compound as an amorphous solid:
NMR ($CDCl_3$): δ 0.53-0.85(m, 4H), 1.37-1.46(m, 1H), 2.60(dt, J = 20.5, 6.5 Hz, 2H), 4.44(dt, J = 46.6, 6.5 Hz, 2H), 5.44(s, 1H), 6.73(d, J=8.4 Hz, 1H), 7.18(dd, J=8.4, 2.3 Hz, 1H), 7.415(d, J=2.2 Hz, 1H), 8.52(s, 1H).

EXAMPLE 17

6-chloro-4-cyclopropyl-4-(3-fluoro-1-propynyl)-3,4-dihydro-3-methylquinazolin-2(1H)-one

A quantity of 100 mg (0.243 mmol) of the product from Example 7 was treated by the procedure of Example 13 to give 29 mg (41%) of the title compound as an amorphous solid:
NMR ($CDCl_3$): δ 0.46-0.57(m, 2H), 0.68-0.84(m, 2H), 1.34-1.42(m, 1H), 3.23(s, 3H), 5.06(d, J=47.2 Hz, 2H), 6.63(d, J=8.4 Hz, 1H), 7.12(s, 1H), 7.20(dd, J=8.4, 2.2 Hz, 1H), 7.397(d, J=2.4 Hz, 1H).

EXAMPLE 18

4-(3-azido-1-propynyl)-6-chloro-4-cyclopropyl-3,4-dihydro-3-methylquinazolin-2(1H)-one

A stirred solution of 50 mg (0.116 mmol) of the product from Example 8 was treated with 38 mg (0.582 mmol) of sodium azide in 0.5 mL dry DMF under Ar for 2 h at room temp. The solvent was removed by rotovap, and the residue partitioned between water and EtOAc. The organic layer was washed with water, brine, dried over $MgSO_4$ and the solvents removed to give an oil which was treated according to the procedure of Example 3 to afford 20 mg (54 %) of the title compound as an amorphous solid:
NMR ($CDCl_3$): δ 0.48-0.58(m, 2H), 0.69-0.77(m, 1H), 0.77-0.85(m, 1H), 1.35-1.43(m, 1H), 3.25(s, 3H), 4.04(s, 2H), 6.79(d, J=8.4 Hz, 1H), 7.18(dd, J=8.4, 2.4 Hz, 1H), 7.39(d, J=2.2 Hz, 1H), 9.23(s, 1H).

EXAMPLE 19

6-chloro-4-cyclopropyl-3,4-dihydro-4-(3-(1-imidazolyl)-1-propynyl)-3-methylquinazolin-2(1H)-one

A stirred solution of 50 mg (0.116 mmol) of the product from Example 8 was treated with 40 mg (0.582 mmol) of imidazole in 1.0 mL of DMF under Ar for 0.5 h at room temp. The reaction was warmed to 50° for 14 hours. The solvent was removed by rotary evaporation, and the residue partitioned between water and $CHCl_3$. The organic layer was washed with brine, dried over $Na_2SO_4$ and the solvents removed to give an oil which was treated by the procedure of Example 3 to afford 15 mg (38 %) of the title compound as an amorphous solid:
NMR ($CDCl_3$): δ 0.40-0.48(m, 1H), 0.51-0.58(m, 1H), 0.67-0.75(m, 2H), 1.34-1.42(m, 1H), 3.20(s, 3H), 4.88(s, 2H), 6.779(d, J=8.4 Hz, 1H), 7.03(s, 1H), 7.14(s, 1H), 7.194(dd, J=8.5, 2.4 Hz, 1H), 7.333(d, J=2.2 Hz, 1H), 7.69(s, 1H), 9.13(s, 1H).

EXAMPLE 20

6-chloro-4-cyclopropyl-3,4-dihydro-3-methyl-4-(3-(2,2,2-trifluoroethoxy)-1-propynyl)quinazolin-2(1H)-one

A solution of 20 mg (0.047 mmol) of the product from Example 8 in 1 mL of $CH_2Cl_2$ was added to stirred mixture of 4.0 µL (0.047 mmol) of trifluoroethanol, 3 mg (0.051 mmol) powdered $K_2CO_3$, and 1µL of tricaprylylmethylammonium chloride under Ar. After stirring for 3 days at room temp., the reaction was diluted with 15 mL of $CHCl_3$ and washed with water, brine, dried over $Na_2SO_4$ and the solvents removed to give an oil which was chromatographed on 3 g fine $SiO_2$ using 1:3 EtOAc-hexane to give an oil which was treated by the procedure of Example 3 to afford 6 mg (13 %) of the title compound as a solid: mp 128-129 °C;
NMR ($CDCl_3$): δ 0.42-0.51(m, 1H), 0.51-0.57(m, 1H), 0.68-0.80(m, 2H), 1.34-1.42(m, 1H), 3.22(s, 3H), 3.90(q, J = 8.6 Hz, 2H), 4.42(s, 2H), 6.645(d, J=8.4 Hz, 1H), 7.191(dd, J=8.4, 2.2 Hz, 1H), 7.23(s, 1H), 7.385(d, J=2.2 Hz, 1H).

EXAMPLE 21

6-chloro-4-cyclopropyl-3,4-dihydro-3-methyl-4-(3-(4-pyridyloxy)-1-propynyl)quinazolin-2(1H)-one

A stirred solution of 50 mg (0.116 mmol) of the product from Example 8 was treated with 96 mg (0.349 mmol) of silver carbonate and 12 mg (0.128 mmol) of 4-hydroxypyridine in 2.0 mL of DMF under Ar for 1.5 h at room temp. The reaction was warmed to 50° overnight. The solvent was removed by rotary evaporation, and the residue partitioned between water and EtOAc. The organic layer was washed with water, brine, dried over $Na_2SO_4$ and the solvents removed to give an oil which was chromatographed on fine $SiO_2$ using 1:3 $CHCl_3$-EtOAc to afford 20 mg of an oil which was treated by the procedure of Example 3 to afford 15 mg ( 17 %) of the title compound as a solid: mp 180-184 °C;
NMR ($CDCl_3$): δ 0.35-0.47(m, 1H), 0.48-0.58(m, 1H), 0.63-0.72(m, 2H), 1.28-1.38(m, 1H), 3.16(s, 3H), 4.90(s, 2H), 6.77(d, J=8.4 Hz, 1H), 6.98(d, J = 6.5 Hz, 2H), 7.18(dd, J=8.4, 2.3 Hz, 1H), 7.29(d, J=2.3 Hz, 1H), 8.55(br d, J = 6 Hz, 2H), 8.78(s, 1H).

EXAMPLE 22

6-chloro-4-cyclopropyl-3,4-dihydro-3-methyl-4-(3-(4-(N-oxopyridyl)oxy)-1-propynyl)quinazolin-2(1H)-one

The title compound (41 mg, 46%) was isolated as a byproduct from Example 21 as a solid: mp 139-141 °C;
NMR ($CDCl_3$ + $CD_3OD$): δ 0.42-0.53(m, 1H), 0.60-0.82(m, 3H), 1.34-1.45(m, 1H), 3.16(s, 3H), 5.07(s, 2H), 6.75(d, J=8.5 Hz, 1H), 6.90-6.97(m, 2H), 7.21(dd, J=8.5, 1.8 Hz, 1H), 7.33(d, J=2.2 Hz, 1H), 8.00(d, J = 7.4 Hz, 2H).

EXAMPLE 23

6-chloro-4-cyclopropyl-3,4-dihydro-1-(4-methoxybenzyl)-4-((2-pyridyl)ethynyl)quinazolin-2(1H)-one

A suspension of 200 mg (0.59 mmol) of 6-chloro-4-cyclopropyl-1-(4-methoxybenzyl)-quinazolin-2(1H)-one (of Example 1, Step C) and 567 mg (1.76 mmol) of magnesium triflate was stirred for 30 min in 10 mL of ether under Ar at rt. In a separate flask, 181 mg (1.76 mmol) of 2-ethynylpyridine was dissolved in 10 mL of dry THF under Ar, cooled to -78 °C, and treated with 704 µL of 2.5 M n-butyllithium in hexanes. After this solution was stirred at -78 °C for 0.5 h, it was added dropwise to the ether suspension described above. The cold bath was removed and stirring continued at room temp. for 2.5 h. A second 1.76 mmol portion of 1-lithio-2-(2-pyridyl)acetylene solution was added to the reaction mixture which was stirred overnight to complete the reaction. The reaction was quenched by pouring into 10% citric acid and extracted with two portions of EtOAc. The organic layers were washed with water, brine, dried over $MgSO_4$ and solvents removed to give an oil which was chromatographed on fine $SiO_2$ using 1:3 hexanes-EtOAc to provide 183 mg (70%) of the title compound as a yellow solid which was used without further purification in the subsequent steps.

EXAMPLE 24

6-chloro-4-cyclopropyl-3,4-dihydro-4-((2-pyridyl)ethynyl)quinazolin-2(1H)-one

A quantity of 70 mg (0.16 mmol) of the product from Example 23 was treated by the procedure of Example 3 for 96 h to afford 38 mg (73 %) of the title compound as an amorphous solid:
NMR ($CDCl_3$): δ 0.58-0.72(m, 1H), 0.73-0.90(m, 2H), 0.91-1.04(m, 1H), 1.47-1.60(m, 1H), 5.85(s, 1H), 6.78(d, J=8 Hz, 1H), 7.15(dd, J=8, 2 HZ, 1H), 7.20-7.28(m, 1H), 7.39(d, J = 8 Hz, 1H), 7.52(d, J=2 Hz, 1H), 7.63-(td, J = 8, 2 Hz, 1H), 8.58(d, J = 4 Hz, 1H), 9.13(s, 1H).

EXAMPLE 25

6-chloro-4-cyclopropyl-3,4-dihydro-4-((3-pyridyl)ethynyl)quinazolin-2(1H)-one

A quantity of 300 mg (0.88 mmol) of 6-chloro-4-cyclopropyl-1-(4-methyoxybenzyl)-quinazolin-2(1H)-one (of Example 1, Step C) was treated with 3-ethynylpyridine (prepared according to Sakamoto et. al., Synthesis, No. 1, p. 312, 1983) by the procedure of Example 23 to afford 187 mg of a yellow solid. A quantity of 100 mg of this material was treated by the procedure of Example 3 to provide 68 mg (39%) of the title compound as a colorless foam. An analytical sample was obtained by trituration with ether-hexane: mp 231-233 °C;
NMR ($CDCl_3$): δ 0.6-0.78(m, 2H), 0.79-0.95(m, 2H), 1.49-1.60(m, 1H), 5.90-6.20(m, 1H), 6.84(d, J=8 Hz, 1H), 7.22(dd, J=8, 2 Hz, 1H), 7.32-7.41(m, 1H), 7.49(s, 1H), 7.76-7.84(m, 1H), 8.40(br s, 1H), 8.50-8.62(m, 1H), 8.75-8.90(m, 1H).

EXAMPLE 26

6-chloro-4-cyclopropyl-3,4-dihydro-4-((4-pyridyl)ethynyl)quinazolin-2(1H)-one

A quantity of 250 mg (0.73 mmol) of 6-chloro-4-cyclopropyl-1-(4-methyoxybenzyl)-quinazolin-2 (1H)-one (of Example 1, Step C) was treated with 4-ethynylpyridine (prepared according to Sakamoto et. al., supra) by the procedure of Example 23 to afford 155 mg of a colorless crystalline solid, mp 157-160 °C. A quantity of 125 mg of this material was treated by the procedure of Example 3 to provide 58 mg (32 %) of the title compound as a solid: mp 131-133 °C;
NMR ($CDCl_3$): δ 0.63-0.76(m, 1H), 0.77-0.81(m, 1H), 0.84-0.96(m, 1H), 0.97-1.04(m, 1H), 1.58-1.63(m, 1H), 5.62(s, 1H), 6.83(d, J=8 Hz, 1H), 7.20-7.40(m, 1H), 7.51(s, 1H), 7.76(s, 1H), 7.80-7.83(m, 2H), 8.74-8.76(m, 2H).

EXAMPLE 27

6-chloro-4-cyclopropyl-3,4-dihydro-4-((2-pyrazinyl)ethynyl)quinazolin-2(1H)-one

A quantity of 120 mg (0.352 mmol) of 6-chloro-4-cyclopropyl-1-(4-methyoxybenzyl)-quinazolin-2(1H)-one (of Example 1, Step C) was treated with 2-ethynylpyrazine (prepared according to Sakamoto et. al., supra) by the procedure of Example 23 as in Step W to afford 110 mg of an oil which was then treated by the procedure of Example 3 to provide 33 mg (29 %) of the title compound as a solid: mp 245 °C (dec);
NMR (DMSO-$d_6$): δ 0.50-0.60(m, 1H), 0.61-0.72(m, 2H), 0.76-0.84(m, 1H), 1.44-1.52(m, 1H), 6.88(d, J=8.6 Hz, 1H), 7.30(dd, J=8.6, 2.4 Hz, 1H), 7.467(d, J=2.4 Hz, 1H), 7.78(d, J=1.7 Hz, 1H), 8.62-8.66(m, 2H), 8.737(d, J = 1.3 Hz, 1H), 9.64(s, 1H).

EXAMPLE 28

6-chloro-4-cyclopropyl-3,4-dihydro-4-((5-pyrimidinyl)ethynyl)quinazolin-2(1H)-one

A quantity of 300 mg ( 0.88 mmol) of 6-chloro-4-cyclopropyl-1-(4-methyoxybenzyl)-quinazolin-2(1H)-one (of Example 1, Step C) was treated with 5-ethynylpyrimidine (prepared according to Sakamoto et. al., supra) according to the procedure of Example 23 to afford 125 mg of a yellow solid, mp 165-167 °C, which was then treated by the procedure of Example 3 to provide 49 mg (18 %) of the title compound as a solid: mp 255-256 °C (dec);

NMR ($CDCl_3$-DMSO-$d_6$): δ 0.60-0.92(m, 4H), 1.47-1.57(m, 1H), 6.54(s, 1H), 6.91(d, J=8.4 Hz, 1H), 7.177(dd, J=8.6, 2.4 Hz, 1H), 7.456(d, J=2.2 Hz, 1H), 7.622(s, 1H), 8.70-8.90(m, 2H), 9.10-9.20(m, 1H), 9.45(s, 1H).

EXAMPLE 29

6-chloro-4-cyclopropyl-3,4-dihydro-3-methyl-4-((2-pyridyl)ethynyl)quinazolin-2(1H)-one

A quantity of 95 mg (0.21 mmol) of the product from Example 23 was treated by the procedure of Example 2 to afford 78 mg of an oil which was then treated according to the procedure of Example 3 to afford 39 mg (54 %) of the title compound as a colorless solid: mp 185.5-186.5 °C;
NMR ($CDCl_3$): δ 0.53-0.65(m, 2H), 0.73-0.80(m, 1H), 0.91-0.98(m, 1H), 1.44-1.51(m, 1H), 3.33(s, 3H), 6.73(d, J=8.4 Hz, 1H), 7.20(dd, J=8.4, 2.2 Hz, 1H), 7.28-7.32(m, 1H), 7.47(d, J = 8 Hz, 1H), 7.51(d, J=2.2 Hz, 1H), 7.697(td, J = 7.7, 1.6 Hz, 1H), 8.3(s, 1H), 8.62-8.64(m, 1H).

EXAMPLE 30

6-chloro-4-cyclopropyl-3,4-dihydro-1-(4-methoxybenzyl)-4-ethynyl)quinazolin-2(1H)-one

A quantity of 5.0 g (14.67 mmol) of 6-chloro-4-cyclopropyl-1-(4-methyoxybenzyl)-quinazolin-2(1H)-one (of Example 1, Step C) was treated with (trimethylsilyl)acetylene according to the procedure of Example 23 to afford approximately 7 g of an oil which was dissolved in 200 mL of THF and stirred vigorously with 150 mL of 1M KOH for 20 min. at room temp. The reaction was acidified with 3M HCl and extracted with two portions of ether. The organic layers were combined and washed with water, brine, dried over $MgSO_4$ and the solvents removed to give an oily yellow solid which was triturated with ether-hexanes, followed by trituration with acetonitrile to afford the title compound as a colorless solid. All of the trituration filtrates were combined, concentrated and retriturated with acetonitrile to give a colorless solid which provided a combined yield of 3.54 g (66 %) of the title compound: NMR ($CDCl_3$): δ 0.59-0.72(m, 2H), 0.77-0.90(m, 2H), 2.52(s, 1H), 3.77(s, 3H), 5.04(d, J = 16.3 Hz, 1H), 5.17(d, J = 16.3 Hz), 5.34(s, 1H), 6.755(d, J = 8.8 Hz, 1H), 6.845(d, J = 8.8Hz, 2H), 7.125(dd, J = 8.8, 2.4 Hz, 1H), 7.193(d, J = 8.79, 2H), 7.50(d, J = 2.4 Hz, 1H).

EXAMPLE 31

6-Chloro-4-cyclopropyl-4-ethynyl-3,4-dihydroquinazolin-2(1H)-one

A solution of 1.4 g (4.11 mmol) of the product from Example 30 in 5 mL of $CH_2Cl_2$ was treated with 10 mL of trifluoroacetic acid under $N_2$ overnight at r.t. The reaction was concentrated by rotary evaporation under reduced pressure and the residue partitioned between ethyl acetate and 10% citric acid. The organic layer was washed with water, brine, dried over $MgSO_4$ and the solvents removed to give an oil which was flash chromatographed on $SiO_2$ using 95:5 $CHCl_3$-$CH_3OH$ to give a foam. Trituration of this material with ether gave 720 mg (80%) of the title compound as a colorless solid.

EXAMPLE 32

6-chloro-4-cyclopropyl-3,4-dihydro-4-((2-pyrimidinyl)ethynyl)quinazolin-2(1H)-one

A mixture of 87 mg (0.35 mmol) of 6-chloro-4-cyclopropyl-3,4-dihydro-4-ethynylquinazolin-2(1H)-one (The product of Example 31), 111 mg (0.7 mmol) of 2-bromopyrimidine, 13 mg (0.018 mmol) of bis-(triphenylphosphine) palladium dichloride, and 1.5 mL of triethylamine was stirred in a sealed tube at 80 °C overnight. After cooling, the reaction was diluted with methanol, filtered through a Celite pad, and concentrated to give an oily solid which was chromatographed on fine $SiO_2$ using 95:5 $CHCl_3$-$CH_3OH$ to provide 75 mg (66%) of the title compound as a colorless solid. An analytical sample was obtained by crystallization from ether-chloroform: mp 259-261 °C (dec);
NMR ($CDCl_3$-DMSO-$d_6$): δ 0.61-0.73 (m, 1H), 0.77-0.87 (m, 2H), 0.95-1.03 (m, 1H), 1.51-1.59 (m, 1H), 6.03 (s, 1H), 6.88 (d, J=8.4 Hz, 1H), 7.169 (dd, J=8.6, 2.4 Hz, 1H), 7.31 (t, J=4.8 Hz, 1H), 7.52 (d, J = 2.2 Hz, 1H), 8.726 (d, J = 4.7 Hz, 2H), 9.27 (s, 1H).

EXAMPLE 33

6-chloro-4-cyclopropyl-3,4-dihydro-3-methyl-4-(3-(N,N-dimethylamino)-1-propynyl)quinazolin-2(1H)-one

A 75 mg (0.175 mmol) sample of the product from Example 8 was treated with 5 mL of dimethylamine (condensed at -78 °C) in a pressure tube. The stirred solution was allowed to warm to room temp. over a 4 h period. After the dimethylamine was allowed to evaporate, the residue was partitioned between $CHCl_3$ and 10% $NaHCO_3$. The organic layer was washed with water, brine, dried over $Na_2SO_4$ and concentrated to give 79 mg of an oil which was treated according to Example 3 to afford 52 mg (93 %) of the title compound as a colorless solid: mp 135-137 °C,
NMR ($CDCl_3$): δ 0.42-0.54 (m, 2H), 0.63-0.77 (m, 1H), 0.78-0.90 (m, 1H), 1.30-1.42 (m, 1H), 2.36 (s, 6H), 3.25 (s, 3H), 3.45 (s, 2H), 6.78 (d, J = 8.4 Hz, 1H), 7.17 (dd, J = 8.4, 2.3 Hz, 1H), 7.395 (d, J = 2.2 Hz, 1H), 9.30 (s, 1H).

EXAMPLE 34

6-Chloro-4-cyclopropyl-3,4-dihydro-4-(phenylethynyl)quinazolin-2(1H)one

A mixture of 70 mg (0.28 mmol) of 6-Chloro-4-cyclopropyl-3,4-dihydro-4-ethynylquinazolin-2(1H)-one (Example 31), was coupled with iodobenzene according to the methods of Example 32 to provide 50 mg of the title compound as a colorless solid: mp 193-195 °C(dec);
NMR ($CDCl_3$): δ 0.59-0.67 (m, 1H), 0.72-0.85 (m, 2H), 0.86-0.98 (m, 1H), 1.48-1.56 (m, 1H), 5.44 (s, 1H), 6.74 (d, J = 8.4 Hz, 1H), 7.21 (dd, J = 8.6, 2.3 Hz, 1H), 7.25-7.40 (m, 5H), 7.51 (d, J = 2.3 Hz, 1H), 8.05 (s,1H).

EXAMPLE 35

4-(3-buten-1-ynyl)-6-chloro-4-cyclopropyl-3,4-dihydro-3-methylquinazolin-2(1H)-one

A solution of 200 mg (0.484 mmol) of the protected, penultimate product of Example 13 (before TFA treatment according to Example 3) was methylated according to Example 2 to give 200 mg of an oil. Treatment of this oil with TFA according to Example 3 provided 20 mg of the title compound as a colorless solid: mp 123-124 °C,
NMR ($CDCl_3$): δ 0.40-0.53 (m, 2H), 0.63-0.73 (m, 1H), 0.73-0.85 (m, 1H), 1.32-1.43 (m, 1H), 3.24 (s, 3H), 5.56-5.93 (m, 3H), 6.77 (d, J = 8.3 Hz, 1H), 7.17 (dd, J = 8.4, 1.9 Hz, 1H), 7.37 (d, J = 1.9 Hz, 1H), 9.19 (s, 1H).

EXAMPLE 36

6-Chloro-4-cyclopropyl-3,4-dihydro-4-(3-hydroxy-1-propynyl)-3-methylquinazolin-2(1H)-one

A sample of 344 mg (0.837 mmol) of the product from Example 7 was deprotected by treatment with TFA according to Example 3, to give 200 mg (82%) of the title compound as a colorless solid. An analytical sample was obtained by crystallization from hexane: mp 76-80 °C.
NMR ($CDCl_3$): δ 0.43-0.53 (m, 2H), 0.65-0.75 (m, 1H), 0.75-0.84 (m, 1H), 1.32-1.39 (m, 1H), 3.22 (s, 3H), 4.39 (s, 2H), 6.68 (d, J = 8.4 Hz, 1H), 7.17 (dd, J = 8.4, 2.4 Hz, 1H), 7.39 (d, J = 2.2 Hz, 1H), 7.90 (s, 1H).

EXAMPLE 37

6-Chloro-4-cyclopropyl-3,4-dihydro-3-methyl-4-(3-(2-pyridyloxy)-1-propynyl)quinazolin-2(1H)-one

A sample of 63 mg (0.129 mmol) of the product from Example 8 and 99.7 mg (1.05 mmol) of 2-hydroxypyridine was treated according to the procedure of Example 21 to give 34 mg (71%) of the title compound as a colorless solid: mp 130-132 °C,
NMR ($CDCl_3$): δ 0.30-0.41 (m, 2H), 0.54-0.65 (m, 1H), 0.65-0.76 (m, 1H), 1.28-1.38 (m, 1H), 3.18 (s, 3H), 5.05 (s, 2H), 6.72 (d, J = 8.4 Hz, 1H), 6.81 (d, J = 8.4 Hz, 1H), 6.92-6.96 (m, 1H), 7.15 (dd, J = 8.4 2.3 Hz, 1H), 7.33 (d, J = 2.2 Hz, 1H), 7.59-7.65 (m, 1H), 8.22 (dd, J = 5.1, 2.0 Hz, 1H), 8.96 (s, 1H).

EXAMPLE 38

6-Chloro-4-cyclopropyl-3,4-dihydro-4-(2-nitrophenylethynyl)quinazolin-2(1H)-one

A mixture of 60 mg (0.24 mmol) of 6-chloro-4-cyclopropyl-3,4-dihydro-4-ethynylquinazolin-2(1H)-one (Example 31), was coupled with 2-iodonitrobenzene according to the procedure of Example 32 to provide 32 mg of the title compound as a colorless solid: mp 181-182 °C(dec);
NMR ($CDCl_3$): δ 0.63-0.71 (m, 1H), 0.77-0.91 (m, 2H), 1.00-1.85 (m, 1H), 1.50-1.58 (m, 1H), 5.51 (s, 1H), 6.76 (d, J = 8.6 Hz, 1H), 7.23 (dd, J = 8.4 2.4 Hz, 1H), 7.45-7.55 (m, 1H), 7.55-7.60 (m, 3H), 8.01 (s, 1H), 8.08 (d, J = 8.05 Hz, 1H).

EXAMPLE 39

1,3-(di-(1S)-camphanoyl)-6-chloro-4-cyclopropyl-3,4-dihydro-4-((2-pyridyl)ethynyl)quinazolin-2(1H)-one

A solution of 200 mg (0.618 mmol) of the product from Example 24, 134 mg (0.618 mmol) of (1S)-camphanic chloride, 76 mg (0.618 mmol) of N,N-dimethylaminopyridine (DMAP), and 0.43 mL (3.09 mmol) of triethylamine in 2.0 mL of $CH_2Cl_2$ was stirred under Ar at rt for 18 hours. An additional 76 mg (0.618 mmol) of DMAP and 268 mg (1.23 mmol) of (1S)-camphanic chloride was added to the reaction mixture, and stirring continued for 6 hours. The reaction was diluted with $CHCl_3$ and washed with 1M citric acid, water, 10% $Na_2CO_3$, dried over $Na_2SO_4$ and treated with activated carbon. Removal of the solvents gave a yellow foam which was chromatographed on 50 g fine $SiO_2$ using 1:2 EtOAc-hexane. The early eluting fractions were combined and evaporated to give 174 mg of diasteromer 1 as an almost colorless foam. Diasteromer 2 was obtained upon further elution as 138 mg of a foam. An analytical sample of diasteromer 2 was obtained by trituration from methanol;

| | | | |
|---|---|---|---|
| Calc'd for $C_{38}H_{38}ClN_3O_7$ | C 66.71, | H 5.60, | N 6.14 |
| Found | C 66.38, | H 5.53, | N 6.17 |

EXAMPLE 40

(-)6-chloro-4-cyclopropyl-3,4-dihydro-4-((2-pyridyl)ethynyl)quinazolin-2(1H)-one

A solution of 143 mg (0.254 mmol) of diasteromer 1 from Example 39 in 1.0 mL of dimethoxyethane was treated with 0.4 mL of 1.0 M aq. LiOH under Ar for 1.5 h. The reaction was partitioned between EtOAc and water. The aqueous layer was extracted with EtOAc and the combined organic layers were washed with water, brine, dried over $MgSO_4$, and the solvents removed to give 115 mg of a foam. This material was dissolved in 2.0 mL of ethanol, treated with 32 mg (0.168 mmol) of p-toluene sulfonic acid and heated at reflux under Ar for 64 h. The solvents were removed by rotary evaporation at reduced pressure and the residue partitioned between 10% $Na_2CO_3$ and EtOAc. The aqueous layer was extracted with EtOAc, and the combined organic layers washed with water, brine, dried over $MgSO_4$, treated with activated carbon, and the solvents removed to give a solid which was triturated with 1:1 $Et_2O$-hexane to provide 16 mg of the title compound as a pale yellow solid: NMR ($CDCl_3$) same as for Example 24; $\alpha_D$ = -100° (c = 0.4, $CHCl_3$).

REVERSE TRANSCRIPTASE ASSAY

The assay measures the incorporation of tritiated deoxyguanosine monophosphate by recombinant HIV reverse transcriptase (HIV $RT_R$) (or other RT) into acid-precipitable cDNA at the Km values of dGTP and poly r(C)•oligo $d(G)_{12-18}$. The inhibitors of the present invention inhibit this incorporation.

The assays were carried out in 55 mM Tris (pH 8.2)-30 mM KCl-30 mM $MgCl_2$-1 mM dithiothreitol-20 μg of $rC:dG_{12-18}$ (Pharmacia) per ml-8 μM [$^3$H]dGTP (New England Nuclear)-0.01% Triton X-100-50 μM ethylene glycol-bis(β-amino-ethyl ether)-N,N,N',N'-tetraacetic acid (EGTA)-1 mg of bovine serum albumin per ml. After 60 min of incubation at 37 °C, acid-precipitable material was collected onto glass fiber filters by using a semiautomatic cell harvester. Bacterial cell extracts containing RT were diluted to within the linear range of the assay, and activity was determined in the presence and absence of inhibitor. Purified HIV-1 RT heterodimer produced in E. coli also served as a control.

For the double (DBL) mutant assay, A17 RT was employed in the assay. A17 RT is resistant to various aminopyridones, as described in Nunberg, J.H. et al., J. Virol. 65, 4887 (1991).

INHIBITION OF VIRUS SPREAD

A. Preparation of HIV-infected MT-4 cell Suspension.

MT cells were infected at Day 0 at a concentration of 250,000 per ml with a 1:1000 dilution of HIV-1 strain IIIb stock (final 125 pg p24/ml; sufficient to yield ≦1% infected cells on day 1 and 25-100% on day 4). Cells were infected and grown in the following medium: RPMI 1640 (Whittaker BioProducts), 10% inactivated fetal bovine serum, 4 mM glutamine (Gibco Labs) and 1:100 Penicillin-Streptomycin (Gibco Labs).

The mixture was incubated overnight at 37°C in 5% $CO_2$ atmosphere.

B. Treatment with Inhibitors

A matrix of nanomolar range concentrations of the pairwise combinations (see Table S) was prepared. At Day 1, aliquots of 125 µl of inhibitors were added to equal volumes of HIV-infected MT-4 cells (50,000 per well) in a 96-well microtiter cell culture plate. Incubation was continued for 3 days at 37°C in 5% $CO_2$ atmosphere.

C. Measurement of Virus Spread

Using a multichannel pipettor, the settled cells were resuspended and 125 µl harvested into a separate microtiter plate. The supernatant was assayed for HIV p24 antigen.

The concentration of HIV p24 antigen was measured by an enzyme immunoassay, described as follows. Aliquots of p24 antigen to be measured were added to microwells coated with a monoclonal antibody specific for HIV core antigen. The microwells were washed at this point, and at other appropriate steps that follow. Biotinylated HIV-specific antibody was then added, followed by conjugated strepavidinhorseradish peroxidase. A color reaction occurs from the added hydrogen peroxide and tetramethylbenzidine substrate. Color intensity is proportional to the concentration of HIV p24 antigen.

Calculation of Degree of Synergy

Pairwise combinations of inhibitors (see Table S) were found to exhibit markedly enhanced inhibition of virus spread, in comparison to each inhibitor alone, or in comparison to merely additive inhibition of each inhibitor. Thus, for example, the pairwise combination of 372 and ddI was found to exhibit markedly enhanced inhibition of virus spread, in comparison to 372 alone or ddI, or in comparison to the sum of 372 inhibitor and ddI inhibition.

This data was processed as follows: fractional inhibitory concentration ratios (FIC) were calculated according to Elion, et. al. J. Biol. Chem., 208, 477 (1954). The minimum sum of FICS, which is the maximum synergy, was determined for various pairwise combinations. See Table S. These results indicate substantial synergy in the inhibition of virus spread. The smaller the number, the greater the synergy.

TABLE S

| Pairwise Combinations* | Maximum Synergy |
|---|---|
| 372 + ddI | 0.3-0.4 |
| 372 + AZT | 0.6-0.8 |
| 372 + 524 | 0.7 |

*372 is 6-chloro-4(S)-cyclopropyl-3,4-dihydro-4-((2-pyridyl)-ethynyl)quinazolin-2(1H)-one, which is compound 26. 524 is L-735,524 (Table C). Other compounds are also defined in Table C above.

While the foregoing specification teaches the principles of the present invention, with examples provided for the purpose of illustration, it will be understood that the practice of the invention encompasses all of the usual variations, adaptations, or modifications, as come within the scope of the following claims and its equivalents.

## Claims

**1.** A compound of the formula :

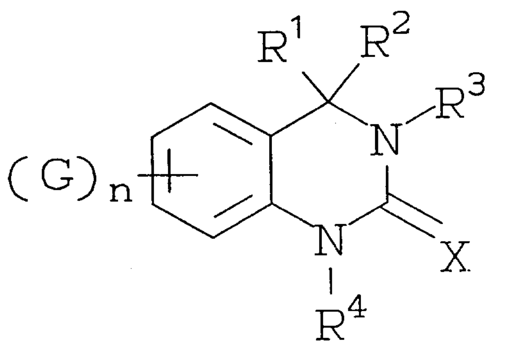

I

wherein:

X is O,

G, when present, is halo; nitro; or cyano;

n is 0-4;

$R^1$ is $C_{3-5}$cycloalkyl; $C_{2-5}$ alkynyl, $C_{2-4}$alkenyl, or cyano;

$R^2$ is $C_{2-5}$alkynyl substituted with one or more of A, or $C_{2-5}$ alkenyl substituted with one or more of A, wherein A is

i) halo,
ii) hydroxy,
iii) amino,
iv) cyano,
v) nitro,
vi) azido,
vii) $C_{3-8}$ cycloalkyl,
viii) $C_{1-4}$ alkoxy, unsubstituted or substituted with one or more of halo,
ix) di-($C_{1-4}$alkyl) amino,
x) $C_{1-4}$ alkylamino,
xi) aryl, unsubstituted or substituted with one or more of D, wherein D is amino, nitro, cyano, or $C_{1-3}$ alkoxy,
xii) aryloxy, unsubstituted or substituted with one or more of D;
xiii) heterocycle, unsubstituted or substituted with one or more of D;
xiv) heterocycle-oxy;
xv) $C_{2-5}$ alkenyl;
xvi) COOR, wherein R is H, $C_{1-4}$alkyl or aryl;
xvii) $CONR_2$; or
xviii) COR;

$R_3$ is

i) H;
ii) cyano;
iii) amino;
iv) hydroxyl;
v) $C_{1-4}$ alkyl, unsubstituted or substituted with one or more of E, wherein E is halo, hydroxyl, amino, nitro, cyano, $C_{1-4}$-alkoxy, or $C_{3-5}$ cycloalkyl;
vi) $C_{2-4}$ alkenyl, unsubstituted or substituted with E; or

vii) $C_{2-4}$ alkynyl, unsubstituted or substituted with E;

$R_4$ is

i) H;
ii) $C_{1-4}$ alkyl;
iii) $C_{1-5}$ alkylcarbonyl;
iv) benzoyl, unsubstituted or substituted with one or more of A; or
v) heterocyclecarbonyl;

with the proviso that any terminal alkynyl carbon is not substituted with any substituent selected from the group consisting of halo, hydroxy, amino, cyano, nitro, azido, $C_{1-4}$alkoxy unsubstituted or substituted with one or more of halo, di-($C_{1-4}$alkyl)amino, $C_{1-4}$alkylamino, aryloxy unsubstituted or substituted with one or more of D, or heterocycle oxy;
or pharmaceutically acceptable salt thereof.

**2.** A compound according to Claim 1, of the formula

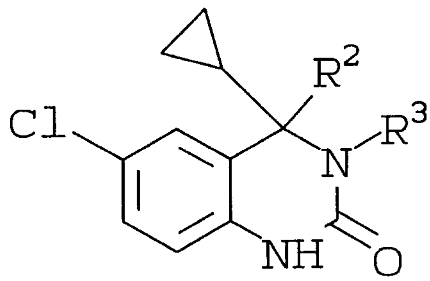

II

wherein:

$R^2$ is $C_{2-5}$ alkynyl substituted with halo, hydroxy, amino, cyano, nitro, azido, $C_{3-8}$ cycloalkyl, $C_{1-4}$ alkoxy, di-($C_{1-4}$ alkyl)amino, $C_{1-4}$ alkylamino, phenyl, 2-nitrophenyl, pyridyl, pyrimidyl, pyrazinyl, imidazolyl, or $C_{2-3}$ alkenyl;
$R^3$ is H or $C_{1-3}$ alkyl;
or pharmaceutically acceptable salt thereof.

**3.** A compound which is
6-chloro-4-cyclopropyl-3,4-dihydro-3-methyl-4-(3-methoxy-1-propynyl)quinazolin-2(1H)-one,
6-chloro-4-cyclopropyl-3,4-dihydro-4-(3-methoxy-1-propynyl)quinazolin-2(1H)-one,
6-chloro-4-cyclopropyl-3,4-dihydro-3-methyl-4-(3-(4-morpholinyl)-1-propynyl)quinazolin-2(1H)-one,
6-chloro-4-cyclopropyl-4-(4-fluoro-1-butynyl)-3,4-dihydro-3-methylquinazolin-2(1H)-one,
6-chloro-4-(4-chloro-1-butynyl)-4-cyclopropyl-3,4-dihydro-3-methylquinazolin-2(1H)-one,
6-chloro-4-cyclopropyl-4-(4-fluoro-1-butynyl)-3,4-dihydroquinazolin-2(1H)-one,
6-chloro-4-cyclopropyl-4-(3-fluoro-1-propynyl)-3,4-dihydro-3-methylquinazolin-2(1H)-one,
4-(3-azido-1-propynyl)-6-chloro-4-cyclopropyl-3,4-dihydro-3-methylquinazolin-2(1H)-one,
6-chloro-4-cyclopropyl-3,4-dihydro-4-(3-(1-imidazolyl)-1-propynyl)-3-methylquinazolin-2(1H)-one,
6-chloro-4-cyclopropyl-3,4-dihydro-3-methyl-4-(3-)2,2,-2,-trifluoroethoxy)-1-propynyl)quinazolin-2(1H)-one,
6-chloro-4-cyclopropyl-3,4-dihydro-3-methyl-4-(3-(4-pyridyloxy)-1-propynyl)quinazolin-2(1H)-one,
6-chloro-4-cyclopropyl-3,4-dihydro-3-methyl-4-(3-(4-(N-oxopyridyl)oxy)-1-propynyl)quinazolin-2(1H)-one,
6-chloro-4-cyclopropyl-3,4-dihydro-4-((2-pyridyl)ethynyl)quinazolin-2(1H)-one,
6-chloro-4-cyclopropyl-3,4-dihydro-4-((3-pyridyl)ethynyl)quinazolin-2(1H)-one,
6-chloro-4-cyclopropyl-3,4-dihydro-4-((4-pyridyl)ethynyl)quinazolin-2(1H)-one,
6-chloro-4-cyclopropyl-3,4-dihydro-4-((2-pyrazinyl)ethynyl)quinazolin-2(1H)-one,
6-chloro-4-cyclopropyl-3,4-dihydro-4-((5-pyrimidinyl)ethynyl)quinazolin-2(1H)-one,
6-chloro-4-cyclopropyl-3,4-dihydro-3-methyl-4-((2-pyridyl)ethynyl)quinazolin-2(1H)-one,
6-chloro-4-cyclopropyl-3,4-dihydro-4-((2-pyrimidinyl)ethynyl)quinazolin-2(1H)-one,
6-chloro-4-cyclopropyl-3,4-dihydro-3-methyl-4-(3-(N,N-dimethylamino)-1-propynyl)quinazolin-2(1H)-one,
6-chloro-4-cyclopropyl-3,4-dihydro-4-(phenylethynyl)quinazolin-2(1H)-one,
4-(3-buten-1-ynyl)-6-chloro-4-cyclopropyl-3,4-dihydro-3-methylquinazolin-2(1H)-one,
6-chloro-4-cyclopropyl-3,4-dihydro-4-(3-hydroxy-1-propynyl)-3-methylquinazolin-2(1H)-one,

6-chloro-4-cyclopropyl-3,4-dihydro-3-methyl-4-(3-(2-pyridyloxy)-1-propynyl)quinazolin-2(1H)-one,
6-chloro-4-cyclopropyl-3,4-dihydro-4-((2-nitrophenyl)ethynyl)quinazolin-2(1H)-one, or
6-chloro-4(S)-cyclopropyl-3,4-dihydro-4-((2-pyridyl)ethynyl)quinazolin-2(1H)-one,
or pharmaceutically acceptable salt thereof.

**4.** A compound of Claim 3, which is
6-chloro-4-cyclopropyl-4-(4-fluoro-1-butynyl)-3,4-dihydro-3-methylquinazolin-2(1H)-one,
6-chloro-4-cyclopropyl-3,4-dihydro-3-methyl-4-((2-pyridyl)ethynyl)quinazolin-2(1H)-one,
6-chloro-4-cyclopropyl-3,4-dihydro-4-((2-pyridyl)ethynyl)quinazolin-2(1H)-one;
6-chloro-4-cyclopropyl-3,4-dihydro-4-(phenylethynyl)quinazoin-2(1H)-one, or
6-chloro-4(S)-cyclopropyl-3,4-dihydro-4-((2-pyridyl)ethynyl)quinazolin-2(1H)-one.
or pharmaceutically acceptable salt thereof.

**5.** The synergistic combination of 6-chloro-4(S)-cyclopropyl-3,4-dihydro-4-((2-pyridyl)ethynyl)quinazolin-2-(1H)-one, and ddI.

**6.** The synergistic combination of 6-chloro-4(S)-cyclopropyl-3,4-dihydro-4-((2-pyridyl)ethynyl)quinazolin-2-(1H)-one, and AZT.

**7.** The synergistic combination of 6-chloro-4(S)-cyclopropyl-3,4-dihydro-4-((2-pyridyl)ethynyl)quinazolin-2-(1H)-one, and N-(2(R)-hydroxy-1(S)-indanyl)-2(R)-phenylmethyl-4-(S)-hydroxy-5-(1-(4-(3-pyridylmethyl)-2(S)-N'-(t-butylcarboxamido)-piperazinyl))-pentanemamide.

**8.** The use of a compound as claimed in any of Claims 1-4 for the manufacture of a medicament for inhibiting HIV reverse transcriptase.

**9.** The use of a compound as claimed in any of Claims 1-4 for the manufacture of a medicament for preventing infection of HIV, or of treating infection by HIV or of treating AIDS or ARC.

**10.** A pharmaceutical composition comprising a pharmaceutically acceptable carrier and an effective amount of a compound as in any of Claims 1-4, or an effective amount of a synergistic combination as in any of claims 5-7.

**11.** A process for the preparation of a compound as claimed in Claim 1, which process comprises reaction of an intermediate of formula (III)

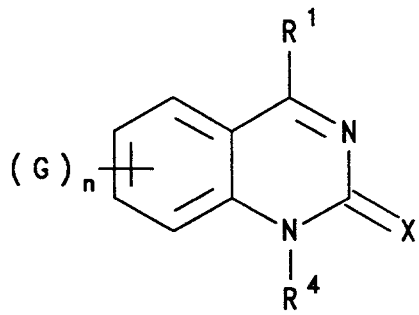

(III)

wherein $R^1$, $R^4$, G, n and X are as defined for formula (I), or a protected derivative thereof, with a reagent suitable to introduce the group $R^2$ or a group convertible thereto, followed, if necessary, by deprotection; and optionally converting the compound of formula (I) so prepared to another compound of formula (I).

European Patent Office

# EUROPEAN SEARCH REPORT

Application Number

EP 93 20 1232

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | GB-A-1 231 959 (SOCIETA FARMACEUTICI ITALIA)<br>* the whole document *<br>--- | 1,10 | C07D239/80<br>A61K31/505<br>C07D401/06<br>C07D403/06<br>C07D405/12<br>C07D401/12<br>A61K31/70 |
| A | CHEMICAL ABSTRACTS, vol. 89, no. 5, 31 July 1978, Columbus, Ohio, US; abstract no. 43474n, page 642 ;<br>* abstract *<br>& JP-A-7 805 180 (SUMITOMO CHEMICAL COMPANY LIMITED) 18 January 1978<br>--- | 1,10 | |
| P,X | EP-A-0 530 994 (MERCK AND CO. INC.)<br>* claims *<br>----- | 1,2,8-11 | |

TECHNICAL FIELDS SEARCHED (Int. Cl.5)

C07D

The present search report has been drawn up for all claims

| Place of search | Date of completion of the search | Examiner |
|---|---|---|
| THE HAGUE | 03 AUGUST 1993 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document